# EUROPÄISCHE PATENTSCHRIFT

(11) **EP 1 194 401 B1**
(45) Veröffentlichungstag und Bekanntmachung des Hinweises auf die Patenterteilung: **10.09.2003**
(21) Anmeldenummer: 00947928.8
(22) Anmeldetag: 05.07.2000
(51) Int. Cl.: C07C 281/06, C07D 213/74, C07C 279/18, C07C 257/14, C07D 233/88, C07C 279/14, A61K 31/197, A61K 31/4402, A61P 9/00, A61P 19/00, A61P 35/00, A61P 31/00

(54) **DIACYLHYDRAZINDERIVATE**
DIACYLHYDRAZINE DERIVATIVES
DERIVES DE DIACYLHYDRAZINE

(30) Priorität: 14.07.1999 DE 19932796
(43) Veröffentlichungstag der Anmeldung: 10.04.2002
(73) Patentinhaber: MERCK PATENT GmbH, 64293 Darmstadt (DE)
(72) Erfinder: HOLZEMANN, Günter, D-64342 Seeheim (DE); GOODMAN, Simon, D-64287 Darmstadt (DE); KESSLER, Horst, D-65824 Schwalbach (DE); GIBSON, Christoph, D-80799 München (DE); SULYOK, Gábor, D-80807 München (DE)
(86) Internationale Anmeldenummer: EP0006307
(87) Internationale Veröffentlichungsnummer: WO01005753

(56) Entgegenhaltungen:
- WO-A-99/20272

## Beschreibung

Die Erfindung betrifft Diacylhydrazinderivate der allgemeinen Formel I 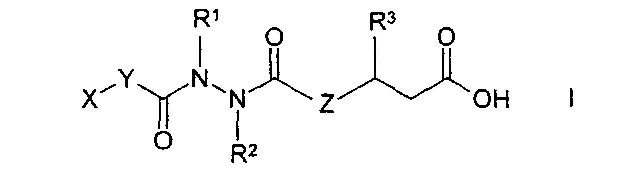 worin
- X: H₂N-C(=NH)-NH-, H₃C-C(=NH)-NH- oder Het¹-NH-,
- Y: -(CH₂)ₙ- oder 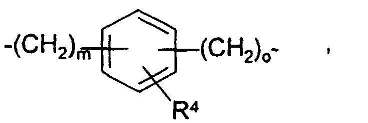
- Z: N-R² oder CH-R²,
- R¹, R²: jeweils unabhängig voneinander H oder A,
- R³: H, Ar oder Het,
- R⁴: H, A, Ar, OH, OA, OAr, Arylalkyl, Hal, CN, NO₂, CF₃ oder OCF₃,
- A: Alkyl mit 1-6 C-Atomen,
- Ar: unsubstituiertes oder ein-, zwei- oder dreifach durch A, OH, OA, CF₃, OCF₃, CN, NO₂ oder Hal substituiertes Phenyl, welches durch ein ein-, zwei- oder dreifach durch A, OH, OA, CF₃, OCF₃, CN, NO₂ oder Hal substituiertes Phenyl in der Art substituiert sein kann, daß ein unsubstituiertes oder substituiertes Biphenyl entsteht oder unsubstituiertes oder ein-, zwei- oder dreifach durch A, OH, OA, CF₃, OCF₃, CN, NO₂ oder Hal substituiertes Naphthyl,
- Hal: F, Cl, Br oder I,
- Het: einen gesättigten, teilweise oder vollständig ungesättigten mono-oder bicyclischen heterocyclischen Rest mit 5 bis 10 Ringgliedern,
wobei 1 oder 2 N- und/oder 1 oder 2 S- oder O-Atome vorliegen können und der heterocyclische Rest ein- oder zweifach durch CN, Hal, OH, OA, CF₃, A, NO₂ oder OCF₃ substituiert sein kann,
- Het¹: einen 5- oder 6-gliedrigen aromatischen Heterocyclus mit 1 bis 4 N-Atomen, der unsubstituiert oder ein- oder zweifach durch Hal, A, OA, Ar, OAr, Arylalkyl, CN, NO₂, CF₃ oder OCF₃ substituiert sein kann,
- n: 2, 3, 4, 5 oder 6,
- m, o: 0, 1 oder 2
bedeuten,
sowie ihre physiologisch unbedenklichen Salze und Solvate.

Teilweise ähnliche Verbindungen sind aus EP 0 632 016, WO 97/26250, WO 97/24124 oder DE 198 31 710 bekannt.

Der Erfindung lag die Aufgabe zugrunde, neue Verbindungen mit wertvollen Eigenschaften aufzufinden, insbesondere solche, die zur Herstellung von Arzneimitteln verwendet werden.

Es wurde gefunden, daß die Verbindungen der Formel I und ihre Salze bei guter Verträglichkeit sehr wertvolle pharmakologische Eigenschaften besitzen. Vor allem wirken sie als Integrin-Inhibitoren, wobei sie insbesondere die Wechselwirkungen der αv-, β3-, β5- oder β6-Integrin-Rezeptoren mit Liganden hemmen, wie z.B. die Bindung von Fibrinogen an den Integrinrezeptor.

Integrine gehören zu der Familie von heterodimeren Klasse I - Transmembran-Rezeptoren, die in zahlreichen Zell-Matrix- bzw. Zell-Zell-Adhäsionsvorgängen eine wichtige Rolle spielen (Tuckwell et al., **1996**, Symp. Soc. Exp. Biol. 47). Sie können grob in drei Klassen eingeteilt werden: die β1-Integrine, die Rezeptoren für die extrazelluläre Matrix darstellen, die β2-Integrine, welche auf Leukozyten aktivierbar sind und während inflammatorischen Prozessen "getriggert" werden, sowie die α**v**-Integrine, die die Zellantwort bei Wundheilungs- und anderen pathologischen Prozessen beeinflussen (Marshall and Hart, **1996**, Semin. Cancer Biol. 7, 191). Die relative Affinität und Spezifität für eine Ligandenbindung wird durch Kombination der verschiedenen α- und β-Untereinheiten bestimmt.

Besondere Wirksamkeit zeigen die erfindungsgemäßen Verbindungen im Fall der Integrine αvβ1, αvβ3, αvβ5, αIIbβ3 sowie αvβ6 und αvβ8, bevorzugt von αvβ3, αvβ5 und αvβ6, sowie αIIbβ3.

αvβ6 ist ein relativ seltenes Integrin (Busk et al., J. Biol. Chem. **1992**, 267(9), 5790), das bei Reparaturvorgängen in Epithelgewebe vermehrt gebildet wird und die natürlichen Matrixmoleküle Fibronectin und Tenascin bevorzugt bindet (Wang et al., Am. J. Respir. Cell Mol. Biol. **1996**, 15(5), 664). Die physiologischen und pathologischen Funktionen von αvβ6 sind noch nicht genau bekannt, es wird jedoch vermutet, daß dieses Integrin bei physiologischen Vorgängen und Erkrankungen (z.B. Entzündungen, Wundheilung, Tumoren), bei denen epitheliale Zellen beteiligt sind, eine wichtige Rolle spielt. So wird αvβ6 auf Keratinozyten in Wunden exprimiert (Haapasalmi et al., J. Invest. Dermatol. **1996**, 106(1), 42), woraus anzunehmen ist, daß neben Wundheilungsprozessen und Entzündungen auch andere pathologische Ereignisse der Haut, wie z. B. Psoriasis, bullöser Pemphigus, Dermatitis und Erytheme sowie auch zystische Fibrose, Endometriose, Leberzirrhose oder Periodontitis durch Agonisten oder Antagonisten des besagten Integrins beeinflußbar sind. Ferner spielt αvβ6 im Atemwegsepithel eine Rolle (Weinacker et al., Am. J. Respir. Cell Mol. Biol. **1995,** *12*(5), 547), so daß entsprechende Agonisten / Antagonisten dieses Integrins bei Atemwegserkrankungen, wie Bronchitis, Asthma, Lungenfibrosen und Atemwegstumoren erfolgreich eingesetzt werden könnten. Letztlich ist bekannt, daß αvβ6 auch im Darmepithel eine Rolle spielt, so daß entsprechende Integrin-Agonisten/-Antagonisten bei der Behandlung von Entzündungen, Tumoren und Wunden des Magen/Darmtraktes Verwendung finden könnten.

Es wurde gefunden, daß die erfindungsgemäßen Verbindungen der Formel I und ihre Salze als lösliche Moleküle Wirkung auf Zellen ausüben, die den genannten Rezeptor tragen, oder wenn sie an Oberflächen gebunden sind, künstliche Liganden für die αvβ6 - vermittelte Zellanhaftung darstellen. Vor allem wirken sie als αvβ6 Integrin-Inhibitoren, wobei sie insbesondere die Wechselwirkungen des Rezeptors mit anderen Liganden hemmen, wie z.B. die Bindung von Fibronectin.

Die erfindungsgemäßen Verbindungen sind insbesondere potente Inhibitoren des Vitronectinrezeptors αvβ3 und/oder potente Inhibitoren des αvβ6-Rezeptors.

Das αvβ3 Integrin wird auf einer Reihe von Zellen, z.B. Endothelzellen, Zellen der glatten Gefäßmuskulatur beispielsweise der Aorta, Zellen zum Abbau von Knochenmatrix (Osteoclasten) oder Tumorzellen, exprimiert.

Die Wirkung der erfindungsgemäßen Verbindungen kann z.B. nach der Methode nachgewiesen werden, die von J.W. Smith et al. in J. Biol. Chem. **1990**, 265, 12267-12271 beschrieben wird. B. Felding-Habermann und D.A. Cheresh beschreiben in Curr. Opin. Cell. Biol. **1993,** 5, 864 die Bedeutungen der Integrine als Adhäsionsrezeptoren für die unterschiedlichsten Phänomene und Krankheitsbilder, speziell in Bezug auf den Vitronectinrezeptor αvβ3.

Die Abhängigkeit der Entstehung von Angiogenese von der Wechselwirkung zwischen vaskulären Integrinen und extrazellulären Matrixproteinen ist von P.C. Brooks, R.A. Clark und D.A. Cheresh in Science **1994**, 264, 569-571 beschrieben.

Die Möglichkeit der Inhibierung dieser Wechselwirkung und damit zum Einleiten von Apoptose (programmierter Zelltod) angiogener vaskulärer Zellen durch ein cyclisches Peptid ist von P.C. Brooks, A.M. Montgomery, M. Rosenfeld, R.A. Reisfeld, T. Hu, G. Klier und D.A. Cheresh in Cell **1994**, 79, 1157-1164 beschrieben. Es wurden darin z.B. αvβ3-Antagonisten oder Antikörper gegen αvβ3 beschrieben, die eine Schrumpfung von Tumoren durch Einleiten von Apoptose bewirken.

Der experimentelle Nachweis, daß auch die erfindungsgemäßen Verbindungen die Anheftung von lebenden Zellen auf den entsprechenden Matrixproteinen verhindern und dementsprechend auch die Anheftung von Tumorzellen an Matrixproteine verhindern, kann in einem Zelladhäsionstest erbracht werden, analog der Methode von F. Mitjans et al., J. Cell Science **1995**, *108*, 2825-2838.

P.C. Brooks et al. beschreiben in J. Clin. Invest. **1995,** *96*, 1815-1822 αᵥβ₃-Antagonisten zur Krebsbekämpfung und zur Behandlung tumor-induzierter angiogener Krankheiten.
Die Verbindungen können die Bindung von Metallproteinasen an Integrine hemmen und so verhindern, daß die Zellen die enzymatische Aktivität der Proteinase nutzen können. Ein Beispiel ist in der Hemmbarkeit der Bindung von MMP-2- (Matrix-Metallo-Proteinase-2-) an den Vitronectin-Rezeptor αvβ3 durch ein Cyclo-RGD-Peptid zu finden, wie in P.C. Brooks et al., Cell **1996**, *85*, 683-693 beschrieben.

Die erfindungsgemäßen Verbindungen der Formel I können daher als Arzneimittelwirkstoffe insbesondere zur Behandlung von Tumorerkrankungen, Osteoporosen, osteolytischen Erkrankungen sowie zur Unterdrückung der Angiogenese eingesetzt werden.

Verbindungen der Formel I, die die Wechselwirkung von Integrinrezeptoren und Liganden, wie z.B. von Fibrinogen an den Fibrinogenrezeptor (Glycoprotein IIb/IIIa) blockieren, verhindern als GPIIb/IIIa-Antagonisten die Ausbreitung von Tumorzellen durch Metastase. Dies wird durch folgende Beobachtungen belegt:
Die Verbreitung von Tumorzellen von einem lokalen Tumor in das vaskuläre System erfolgt durch die Bildung von Mikroaggregaten (Mikrothromben) durch die Wechselwirkung der Tumorzellen mit Blutplättchen. Die Tumorzellen sind durch den Schutz im Mikroaggregat abgeschirmt und werden von den Zellen des Immunsystems nicht erkannt. Die Mikroaggregate können sich an Gefäßwandungen festsetzen, wodurch ein weiteres Eindringen von Tumorzellen in das Gewebe erleichtert wird. Da die Bildung der Mikrothromben durch Fibrinogenbindung an die Fibrinogenrezeptoren auf aktivierten Blutplättchen vermittelt wird, können die GPIIb/IIIa-Antagonisten als wirksame Metastase-Hemmer angesehen werden.

Verbindungen der Formel I hemmen neben der Bindung von Fibrinogen, Fibronectin und des von-Willebrand-Faktors an den Fibrinogenrezeptor der Blutplättchen auch die Bindung weiterer adhäsiver Proteine, wie Vitronectin, Kollagen und Laminin, an die entsprechenden Rezeptoren auf der Oberfläche verschiedener Zelltypen. Sie verhindern insbesondere die Entstehung von Blutplättchenthromben und können daher zur Behandlung von Thrombosen, Apoplexie, Herzinfarkt, Entzündungen und Arteriosklerose eingesetzt werden.

Die thrombozytenaggregationshemmende Wirkung läßt sich in vitro nach der Methode von Born (Nature **1962**, *4832*, 927-929) nachweisen.

Ein Maß für die Aufnahme eines Arzneimittelwirkstoffs in einen Organismus ist seine Bioverfügbarkeit.
Wird der Arzneimittelwirkstoff in Form einer Injektionslösung dem Organismus intravenös zugefügt, so liegt seine absolute Bioverfügbarkeit, d.h. der Anteil des Pharmakons, der unverändert im systemischen Blut, d.h. in den großen Kreislauf gelangt, bei 100%.
Bei oraler Vergabe eines therapeutischen Wirkstoffs liegt der Wirkstoff in der Regel als Feststoff in der Formulierung vor und muß sich daher zuerst auflösen, damit er die Eintrittsbarrieren, beispielsweise den Gastrointestinaltrakt, die Mundschleimhaut, nasale Membranen oder die Haut, insbesondere das Stratum corneum, überwinden kann bzw. vom Körper resorbiert werden kann. Daten zur Pharmakokinetik, d.h. zur Bioverfügbarkeit können analog zu der Methode von J. Shaffer et al, J. Pharm. Sciences, 1999, 88, 313-318 erhalten werden.

Gegenstand der Erfindung sind Verbindungen der Formel I nach Anspruch 1 und ihre physiologisch unbedenklichen Salze und/oder Solvate als therapeutische Wirkstoffe.

Gegenstand der Erfindung sind demgemäß Verbindungen der Formel I nach Anspruch 1 und ihre physiologisch unbedenklichen Salze und/oder Solvate als Integrininhibitoren.

Gegenstand der Erfindung sind Verbindungen der Formel I nach Anspruch 1 und ihre physiologisch unbedenklichen Salze und/oder Solvate zur Anwendung bei der Bekämpfung von Krankheiten.

Die Verbindungen der Formel I können als Arzneimittelwirkstoffe in der Human- und Veterinärmedizin eingesetzt werden, insbesondere zur Prophylaxe und/oder Therapie von Erkrankungen des Kreislaufs, Thrombose, Herzinfarkt, Arteriosklerose, Apoplexie, Angina pectoris, Tumorerkrankungen, wie Tumorwachstum oder Tumormetastasierung, osteolytischen Krankheiten wie Osteoporose, pathologisch angiogenen Krankheiten wie z.B. Entzündungen, ophthalmologischen Krankheiten, diabetischer Retinopathie, makularer Degeneration, Myopia, okularer Histoplasmose, rheumatischer Arthritis, Osteoarthritis, rubeotischem Glaukom, ulcerativer Colitis, Morbus Crohn, Atherosklerose, Psoriasis, bullöser Pemphigus, Dermatitis, Erytheme, Lungenfibrose, zystische Fibrose, Endometriose, Leberzirrhose, Periodontitis, Restenose nach Angioplastie, Multiplesklerose, viraler Infektion, bakterieller Infektion, Pilzinfektion, bei akutem Nierenversagen und bei der Wundheilung zur Unterstützung des Heilungsprozesses.

Die Verbindungen der Formel I können als antimikrobiell wirkende Substanzen bei Operationen eingesetzt werden, wo Biomaterialien, Implantate, Katheter oder Herzschrittmacher verwendet werden.
Dabei wirken sie antiseptisch. Die Wirksamkeit der antimikrobiellen Aktivität kann durch das von P. Valentin-Weigund et al. in Infection and Immunity, **1988**, 2851-2855 beschriebene Verfahren nachgewiesen werden.

Da die Verbindungen der Formel I Inhibitoren der Fibrinogenbindung und damit Liganden der Fibrinogenrezeptoren auf Blutplättchen darstellen, können sie als Diagnostika zur Detektion und Lokalisierung von Thromben im vaskulären System *in vivo* verwendet werden, sofern sie beispielsweise durch einen radioaktiven oder UV-detektierbaren Rest substituiert werden.

Die Verbindungen der Formel I können als Inhibitoren der Fibrinogenbindung auch als wirksame Hilfsmittel zum Studium des Metabolismus von Blutplättchen in unterschiedlichen Aktivierungsstadien oder von intrazellulären Signalmechanismen des Fibrinogenrezeptors verwendet werden. Die detektierbare Einheit eines einzubauenden "Labels", z.B. eine Isotopenmarkierung durch ³H, erlaubt es, nach Bindung an den Rezeptor, die genannten Mechanismen zu untersuchen.

Es bedeuten nachstehend:
- Ac: Acetyl
- Aza-Gly: H₂N-NH-COOH
- BOC: tert.-Butoxycarbonyl
- CBZ oder Z: Benzyloxycarbonyl
- DCCI: Dicyclohexylcarbodiimid
- DCM: Dichlormethan
- DIPEA: Diisopropylethylamin
- DMF: Dimethylformamid
- EDCI: N-Ethyl-N,N'-(dimethylaminopropyl)-carbodiimid
- Et: Ethyl
- Fmoc: 9-Fluorenylmethoxycarbonyl
- Gly: Glycin
- HATU: O-(7-Azabenzotriazol-1-yl)-N,N,N',N'-tetramethyluroniumhexafluorophosphat
- HOBt: 1-Hydroxybenzotriazol
- Me: Methyl
- MBHA: 4-Methyl-benzhydrylamin
- Mtr: 4-Methoxy-2,3,6-trimethylphenyl-sulfonyl
- NMP: N-Methylpyrrolidon
- NMR: kernmagnetische Resonanz
- HONSu: N-Hydroxysuccinimid
- OBzl: Benzylester
- OtBu: tert.-Butylester
- Oct: Octanoyl
- OMe: Methylester
- OEt: Ethylester
- Pbf: 2,2,4,6,7-Pentamethyldihydrobenzofuran-5-sulfonyl
- β-Phe: β-Phenylalanin
- POA: Phenoxyacetyl
- R_{f}-Wert: Retentionsfaktor
- RP: *Reversed* Phase
- RT: Retentionszeit
- Sal: Salicyloyl
- TBTU: O-(1 H-Benzotriazol-1-yl)-N,N,N',N'-tetramethyluroniumtetrafluorborat
- TFA: Trifluoressigsäure
- Trt: Trityl (Triphenylmethyl).

Die Verbindungen der Formel I besitzen mindestens ein chirales Zentrum und können daher in mehreren stereoisomeren Formen auftreten. Alle diese Formen (z. B. D- und L-Formen) und deren Gemische (z. B. die DL-Formen) sind in der Formel I eingeschlossen.

In die erfindungsgemäßen Verbindungen nach Anspruch 1 sind auch sogenannte Prodrug-Derivate eingeschlossen, d.h. mit z.B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. **1995**, *115*, 61-67 beschrieben ist.

In die erfindungsgemäßen Verbindungen nach Anspruch 1 sind auch Derivate der Verbindungen der Formel I eingeschlossen, deren Carboxylgruppe in einen pharmazeutisch akzeptablen metabolisch labilen Ester oder ein Amid davon umgewandelt ist.

Ferner können freie Aminogruppen oder freie Hydroxygruppen als Substituenten von Verbindungen der Formel I mit entsprechenden Schutzgruppen versehen sein.

Unter Solvaten der Verbindungen der Formel I werden Anlagerungen von inerten Lösungsmittelmolekülen an die Verbindungen der Formel I verstanden, die sich aufgrund ihrer gegenseitigen Anziehungskraft ausbilden. Solvate sind z.B. Mono- oder Dihydrate oder Additionsverbindungen mit Alkoholen, wie z.B. mit Methanol oder Ethanol.

Gegenstand der Erfindung ist ferner ein Verfahren zur Herstellung von Verbindungen der Formel I nach Anspruch 1 sowie ihrer Salze, dadurch gekennzeichnet, daß man
a) zur Herstellung einer Verbindung der Formel I, worin Z N-R² bedeutet, eine Verbindung der Formel II, 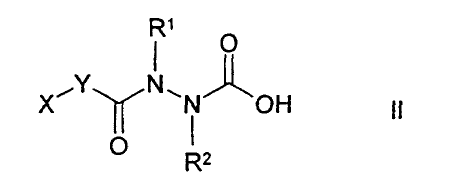 worin X, Y, R¹ und R² die in Anspruch 1 angegebenen Bedeutungen haben, und worin freie Aminogruppen durch eine geeignete Aminoschutzgruppe geschützt sind,
   mit einer Verbindung der Formel III 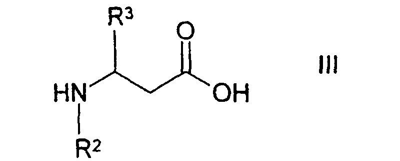 worin R² und R³ die in Anspruch 1 angegebenen Bedeutungen haben und worin eine freie Hydroxylgruppe durch eine geeignete Hydroxylschutzgruppe geschützt ist oder an eine feste Phase gebunden ist,
   umsetzt,
   und anschließend die Schutzgruppen und/oder die feste Phase entfernt,
   oder
b) eine Verbindung der Formel IV, 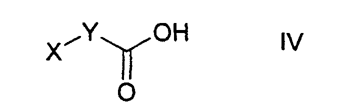 worin X und Y die in Anspruch 1 angegebenen Bedeutungen haben, und worin freie Aminogruppen durch eine geeignete Aminoschutzgruppe geschützt sind,
   mit einer Verbindung der Formel V 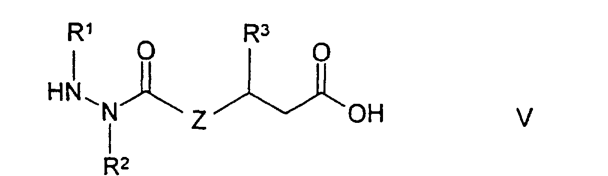 worin R¹, R², R³ und Z die in Anspruch 1 angegebenen Bedeutungen haben, und worin eine freie Hydroxylgruppe durch eine geeignete Hydroxylschutzgruppe geschützt ist oder an eine feste Phase gebunden ist,
   umsetzt,
   und anschließend die Schutzgruppen und/oder die feste Phase entfernt;
   oder
c) eine Verbindung der Formel I aus einem ihrer funktionellen Derivate durch Behandeln mit einem solvolysierenden oder hydrogenolysierenden Mittel in Freiheit setzt,
   und/oder daß man eine basische oder saure Verbindung der Formel I durch Behandeln mit einer Säure oder Base in eines ihrer Salze überführt.

Für die gesamte Erfindung gilt, daß sämtliche Reste, die mehrfach auftreten, wie z.B. R² gleich oder verschieden sein können, d.h unabhängig voneinander sind.

In den vorstehenden Formeln bedeutet A Alkyl, ist linear oder verzweigt, und hat 1 bis 6, vorzugsweise 1, 2, 3, 4, 5 oder 6 C-Atome. A bedeutet vorzugsweise Methyl, weiterhin Ethyl, Isopropyl, n-Propyl, n-Butyl, Isobutyl, sek.-Butyl oder tert.-Butyl, ferner auch n-Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1-oder 2-Ethylbutyl, 1-Ethyl-1-methylpropyl, 1-Ethyl-2-methylpropyl, 1,1,2- oder 1,2,2-Trimethylpropyl.
Besonders bevorzugt für A ist Methyl.

Aminoschutzgruppe bedeutet vorzugsweise Acetyl, Propionyl, Butyryl, Phenylacetyl, Benzoyl, Toluyl, POA, Methoxycarbonyl, Ethoxycarbonyl, 2,2,2-Trichlorethoxycarbonyl, BOC, 2-lodethoxycarbonyl, CBZ ("Carbobenzoxy"), 4-Methoxybenzyloxycarbonyl, Fmoc, Mtr oder Benzyl. Besonders bevorzugt Fmoc.

Ar bedeutet vorzugsweise unsubstituiertes Phenyl, weiterhin vorzugsweise durch A, OH, OA, CF₃, OCF₃, CN, NO₂ oder Hal mono-, di- oder trisubstituiertes Phenyl, welches ebenfalls durch ein ein-, zwei- oder dreifach durch A, OH, OA, CF₃, OCF₃, CN, NO₂ oder Hal substituiertes Phenyl in der Art substituiert sein kann, daß ein unsubstituiertes oder substituiertes Biphenyl entsteht. A und Hal haben die zuvor oder nachstehend angeführten bevorzugten oder besonders bevorzugten Bedeutungen.
Ar bedeutet weiterhin vorzugsweise unsubstituiertes oder ein-, zwei- oder dreifach durch A, OH, OA, CF₃, OCF₃, CN, NO₂ oder Hal substituiertes Naphthyl, wobei A und Hal eine der zuvor oder nachstehend angeführten bevorzugten oder besonders bevorzugten Bedeutungen hat.

Ar bedeutet daher bevorzugt Phenyl, o-, m- oder p-Methylphenyl, o-, m-oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-, m- oder p-Ethoxyphenyl, o-, m-, p-Trifluormethylphenyl, o-, m-, p-Trifluormethoxyphenyl, o-, m- oder p-Fluorphenyl, o-, m- oder p-Chlorphenyl, o-, m- oder p-Bromphenyl, o-, m-, p-Nitrophenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl, 2,3-, 2,4-, 2,5-, 2,6-, 3,4- oder 3,5-Dihydroxyphenyl, 2,3-, 2,4-, 2,5-, 2,6-, 3,4-oder 3,5-Ditrifluormethylphenyl, 2,3-, 2,4-, 2,5-, 2,6-, 3,4- oder 3,5-Ditrifluormethoxyphenyl, 2,3-, 2,4-, 2,5-, 2,6-, 3,4- oder 3,5-Dinitrophenyl, 2,3-, 2,4-, 2,5-, 2,6-, 3,4- oder 3,5-Difluorphenyl, 2,3-, 2,4-, 2,5-, 2,6-, 3,4-oder 3,5-Dichlorphenyl, 2,3-, 2,4-, 2,5-, 2,6-, 3,4- oder 3,5-Dibromphenyl. Ferner aber auch bevorzugt unsubstituiertes Biphenyl oder auch substituiertes Biphenyl, im einzelnen bevorzugt 4-Biphenyl oder 3-Biphenyl, 4'-(2-Methyl-biphenyl), 4'-(3-Methyl-biphenyl), 4'-(4-Methyl-biphenyl), 3'-(2-Methyl-biphenyl), 3'-(3-Methyl-biphenyl), 3'-(4-Methyl-biphenyl), 4-(2-Methyl-biphenyl), 4-(3-Methyl-biphenyl), 3-(2-Methyl-biphenyl), 3-(4-Methylbiphenyl), 4'-(2-Fluor-biphenyl), 4'-(3-Fluor-biphenyl), 4'-(4-Fluor-biphenyl), 3'-(2-Fluor-biphenyl), 3'-(3-Fluor-biphenyl), 3'-(4-Fluor-biphenyl), 4-(2-Fluorbiphenyl), 4-(3-Fluor-biphenyl), 3-(2-Fluor-biphenyl) oder 3-(4-Fluorbiphenyl).
Femer aber auch bevorzugt unsubstituiertes Naphthalin-1-yl oder Naphthalin-2-yl.

Besonders bevorzugt bedeutet Ar Phenyl, p-Chlorphenyl, p-Bromphenyl, p-Fluorphenyl, 3,5-Dichlorphenyl, o- oder m-Nitrophenyl, p-Trifluormethoxyphenyl, p-Methoxyphenyl, 3,5-Dichlor-2-hydroxy-phenyl, Naphthalin-1-yl, Biphenyl-4-yl oder 4'-(4-Fluor-biphenyl).

Arylalkyl bedeutet bevorzugt Benzyl, Phenylethyl, Phenylpropyl oder Naphthylmethyl, besonders bevorzugt Benzyl.

Hal bedeutet vorzugsweise F, Cl oder Br.

Het bedeutet einen gesättigten, teilweise oder vollständig ungesättigten mono- oder bicyclischen heterocyclischen Rest mit 5 bis 10 Ringgliedern,
wobei 1 oder 2 N- und/oder 1 oder 2 S- oder O-Atome vorliegen können und der heterocyclische Rest ein- oder zweifach durch CN, Hal, OH, OA, CF₃, A, NO₂ oder OCF₃ substituiert sein kann.

Het ist vorzugsweise substituiertes oder unsubstituiertes 2- oder 3-Furyl, 2-oder 3-Thienyl, 1-, 2- oder 3-Pyrrolyl, 1-, 2-, 4- oder 5-Imidazolyl, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder-5-yl, 1,2,4-Triazol-1-, -4- oder -5-yl, 1- oder 5-Tetrazolyl, 1,2,3-Oxadiazol-4- oder -5-yl 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4H-Thiopyranyl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-1H-Indolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 1-, 2-, 3-, 4-, 5-, 6-, 7- oder 8-Chinolinyl, 1-, 3-, 4-, 5-, 6-, 7- oder 8-Isochinolinyl, 1-, 2-, 3-, 4- oder 9-Carbazolyl, 1-, 2-, 3-, 4-, 5-, 6-, 7-, 8- oder 9-Acridinyl, 3-, 4-, 5-, 6-, 7- oder 8-Cinnolinyl, 2-, 4-, 5-, 6-, 7-oder 8-Chinazolinyl. Die heterocyclischen Reste können auch teilweise oder vollständig hydriert sein. Het kann also auch bedeuten 2,3-Dihydro-2-, -3-, -4- oder -5-furyl, 2,5-Dihydro-2-, -3-, -4- oder -5-furyl, Tetrahydro-2-oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -3-pyrollyl, Tetrahydro-1-,-2- oder 4-imidazolyl, 2,3-Dihydro-1-, -2-, -3-, -4-, -5-, -6-, -7-1H-indolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1,2,3,6-Tetrahydro-1-, -2-, -3, -4-, -5- oder -6-pyridyl, 1-, 2-, 3- oder 4-Piperidinyl, 1-, 2-, 3- oder 4-Azepanyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-,-4- oder -5-pyrimidinyl, 1-, 2- oder 3- Piperazinyl, 1,2,3,4-Tetrahydro-1-, -2-, -3-, -4-, -5-, -6-, -7- oder -8-chinolinyl, 1,2,3,4-Tetrahydro-1-, -2-, -3-, -4-, -5-, -6-, -7- oder -8-isochinolinyl.
Besonders bevorzugt ist Het 2-, 3- oder 4-Pyridyl oder 1-, 2-, 3-, 4-, 5-, 6-oder 7-1H-Indolyl.

Het¹ bedeutet einen 5- oder 6-gliedrigen aromatischen Heterocyclus mit 1 bis 4 N-Atomen, der unsubstituiert oder ein- oder zweifach durch Hal, A, OA, Ar, OAr, Arylalkyl, CN, NO₂, CF₃ oder OCF₃ substituiert sein kann,
wobei Hal, A, Arylalkyl und Ar eine der zuvor angegebenen Bedeutungen hat.

Het¹ ist vorzugsweise unsubstituiertes oder substituiertes 2- oder 3-Pyrrolyl, 2, 4- oder 5-Imidazolyl, 3-, 4- oder 5-Pyrazolyl, 2-, 3- oder 4-Pyridyl, 2-, 4-, 5- oder 6-Pyrimidinyl, 3- oder 4-Pyridazinyl, 2- oder 3-Pyrazinyl.
Besonders bevorzugt ist Pyridin-2-yl, Imidazol-2-yl oder 4-Methyl-pyridin-2-yl.

Het¹-NH ist vorzugsweise Pyrrol-2- oder Pyrrol-3-ylamin, Imidazol-2-, lmidazol-4- oder Imidazol-5-ylamin, Pyrazol-3-, Pyrazol-4- oder Pyrazol-5-ylamin, Pyridyl-2-, Pyridyl-3- oder Pyridyl-4-ylamin, Pyrimidin-2-, Pyrimidin-4-, Pyrimidin-5- oder Pyrimidin-6-ylamin, Pyridazin-3- oder Pyridazin-4-ylamin, Pyrazin-2- oder Pyrazin-3-ylamin, wobei die erwähnten heterocyclischen Ringe durch bevorzugt Alkyl substituiert sein können. Besonders bevorzugt für Het¹-NH ist Pyridyl-2-ylamin, Imidazol-2-ylamin oder 4-Methyl-pyridin-2-ylamin.

OA bedeutet vorzugsweise Methoxy, Ethoxy, Propoxy oder Butoxy, ferner auch Pentyloxy oder Hexyloxy.

R¹ bedeutet vorzugsweise H oder A, wobei A eine der zuvor angegebenen Bedeutungen hat; insbesondere H.

R² bedeutet vorzugsweise H oder A, wobei A eine der zuvor angegebenen Bedeutungen hat; insbesondere H.

R³ bedeutet vorzugsweise H, Ar oder Het, wobei Ar und Het eine der zuvor angegebenen Bedeutungen haben; insbesondere H, Phenyl, p-Chlorphenyl, p-Bromphenyl, p-Fluorphenyl, 3,5-Dichlorphenyl, o- oder m-Nitrophenyl, p-Trifluormethoxyphenyl, p-Methoxyphenyl, 3,5-Dichlor-2-hydroxy-phenyl, Naphthalin-1-yl, Biphenyl-4-yl oder 4'-(4-Fluor-biphenyl).

R⁴ bedeutet vorzugsweise H, A, Ar, OH, OA, OAr, Arylalkyl, Hal, CN, NO₂, CF₃ oder OCF₃, wobei A, Arylalkyl, Ar und Hal eine der zuvor angegebenen Bedeutungen haben; insbesondere H und Methyl.

X bedeutet H₂N-C(=NH)-NH-, H₃C-C(=NH)-NH- oder Het¹-NH, wobei Het¹-NH eine der zuvor angegebenen Bedeutungen hat; insbesondere bedeutet X H₂N-C(=NH)-NH-, H₃C-C(=NH)-NH-, Pyridin-2-ylamino, Imidazol-2-ylamino oder 4-Methyl-pyridin-2-ylamino.

Y bedeutet -(CH₂)ₙ- oder 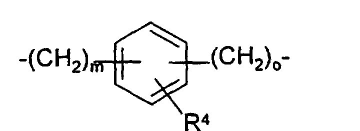 wobei n 2, 3, 4, 5 oder 6, insbesondere 3 oder 4 bedeutet und m 0, 1 oder 2, insbesondere 0 und o 0, 1 oder 2, insbesondere 0 oder 1 bedeuten und R⁴ eine der zuvor angegebenen Bedeutungen hat.

Z bedeutet N-R² oder CH-R², wobei R² eine der zuvor angegebenen Bedeutungen hat, insbesondere NH oder CH₂.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis Ir ausgedrückt werden, die der Formel I entsprechen und worin die nicht näher bezeichneten Reste die bei der Formel I angegebene Bedeutung haben, worin jedoch
- in la): X H₂N-C(=NH)-NH-bedeutet,
- in lb): Y 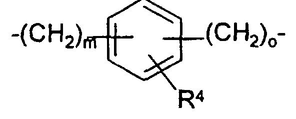 bedeutet,
- in lc): Z N-R₂ bedeutet,
- in ld): Z CH-R₂ bedeutet,
- in le): X H₂N-C(=NH)-NH-, H₃C-C(=NH)-NH- oder Het¹-NH-und
Y -(CH₂)ₙ-bedeutet,
- in lf): X H₂N-C(=NH)-NH-und 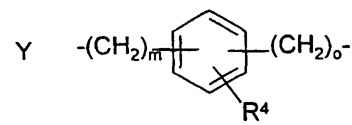 bedeutet,
- in lg): X H₂N-C(=NH)-NH- oder Het¹-NH-,
Y -(CH₂)ₙ-,
R³ Ar,
Z NH beteutet,
- in lh): X H₂N-C(=NH)-NH-,
R³ Ar,
Z NH und, 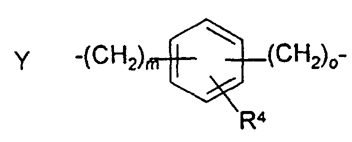 beteutet,
- in li): X H₂N-C(=NH)-NH- oder Het¹-NH-,
R³ Ar,
Y -(CH₂)ₙ- und,
Z CH₂ bedeutet,
- in lk): X H₂N-C(=NH)-NH-
R³ Ar,
Z CH₂ und 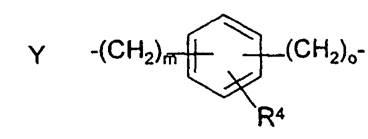 bedeutet,
- in lm): X H₂N-C(=NH)-NH-, H₃C-C(=NH)-NH-,
Y -(CH₂)ₙ- oder 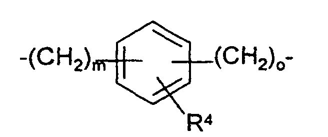
Z NH oder CH₂,
R¹,R² H,
R³ Ar,
R⁴ H,
Het¹ 4-Methyl-pyridin-2yl, Pyridin-2-yl oder Imidazol-2yl,
n 3 oder 4,
m 0 und
o 0 oder 1 bedeutet,
- in ln): X H₂N-C(=NH)-NH- oder Het¹-NH-,
Y -(CH₂)ₙ- oder 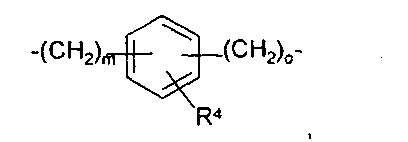
Z NH,
R¹,R² H,
R³ unsubstituiertes oder ein-, zwei- oder dreifach durch OH, OA, OCF₃, NO₂ oder Hal substituiertes Phenyl, welches durch ein unsubstituiertes oder ein ein-, zwei- oder dreifach durch Hal substituiertes Phenyl in der Art substituiert sein kann, daß ein unsubstituiertes oder substituiertes Biphenyl entsteht oder unsubstituiertes Naphthyl,
R⁴ H,
Het¹ 4-Methyl-pyridin-2-yl, Pyridin-2-yl oder Imidazol-2-yl,
n 3 oder 4, und
m 0 und
o 0 oder 1 bedeutet,
- in lo): X H₂N-C(=NH)-NH- oder Het¹-NH-,
Y -(CH₂)ₙ- oder 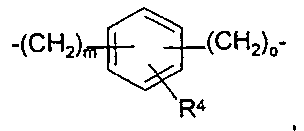
Z CH₂,
R¹,R² H,
R⁴ H,
R³ unsubstituiertes oder ein-, zwei- oder dreifach durch OH, OA, OCF₃, NO₂ oder Hal substituiertes Phenyl, welches durch ein unsubstituiertes oder ein ein-, zwei- oder dreifach durch Hal substituiertes Phenyl in der Art substituiert sein kann, daß ein unsubstituiertes oder substituiertes Biphenyl entsteht oder unsubstituiertes Naphthyl,
Het¹ 4-Methyl-pyridin-2-yl, Pyridin-2-yl oder Imidazol-2-yl,
n 4 und
m,o 0 bedeutet,
- in lp): X H₂N-C(=NH)-NH- oder Het¹ -NH-
Y -(CH₂)ₙ- oder 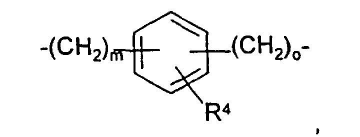
Z NH oder CH₂,
R¹,R² H,
R⁴ H,
R³ unsubstituiertes oder ein-, zwei- oder dreifach durch OH, OA, OCF₃, NO₂ oder Hal substituiertes Phenyl, welches durch ein unsubstituiertes oder ein ein-, zwei- oder dreifach durch Hal substituiertes Phenyl in der Art substituiert sein kann, daß ein unsubstituiertes oder substituiertes Biphenyl entsteht oder unsubstituiertes Naphthyl,
Het¹ 4-Methyl-pyridin-2-yl, Pyridin-2-yl oder Imidazol-2-yl,
n 3 oder 4,
m 0 und
o 0 oder 1 bedeutet,
- in lq): R³ unsubstituiertes Biphenyl-4-yl oder ein-, zwei-oder dreifach durch Hal substituiertes Phenyl,
- in lr): X H₂N-C(=NH)-NH- oder Het¹-NH-,
Y -(CH₂)ₙ- oder 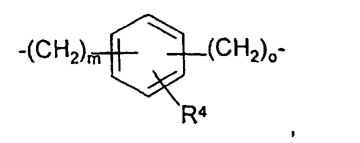
Z NH oder CH₂,
R¹,R² H,
R⁴ H,
R³ Biphenyl-4-yl, 4-Chlorphenyl oder 3.5-Dichlorphenyl,
Het¹ 4-Methyl-pyridin-2yl, Pyridin-2yl oder Imidazol-2-yl,
n 3 oder 4,
m 0 und
o 0 oder 1 bedeutet.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Verbindungen der Formel I können vorzugsweise unter den Bedingungen einer Peptidsynthese erhalten werden. Dabei arbeitet man zweckmäßig nach üblichen Methoden der Peptidsynthese, wie sie z.B. in Houben-Weyl, 1.c., Band 15/II, Seite 1 bis 806 (1974) beschrieben sind.

Die direkten Vorstufen der Verbindungen der Formel I können auch z.B. nach Merrifield (Angew. Chem. 97, 801-812, 1985) an einer festen Phase, z.B. einem quelifähigen Polystyrolharz, aufgebaut werden. Als feste Phase können prinzipiell alle Träger, wie sie z.B. aus der Festphasen-Peptidchemie oder Nucleinsäuresynthese bekannt sind, verwendet werden.
Als polymere Trägermaterialien eignen sich polymere feste Phasen mit vorzugsweise hydrophilen Eigenschaften, beispielsweise quervernetzte Polyzucker wie Cellulose, Sepharose oder Sephadex^{R}, Acrylamide, Polymer auf Polyethylenglykolbasis oder Tentakelpolymere^{R}. Vorzugsweise wird als feste Phase Tritylchloridpolystyrol-Harz, 4-Methoxytritylchlorid-Harz, Merrifield-Harz,und Wang-Harz eingesetzt.

So können Verbindungen der Formel I erhalten werden, indem man eine Verbindung der Formel II mit einer Verbindung der Formel III umsetzt, und anschließend die Schutzgruppen oder die feste Phase entfernt.

Die Verbindungen der Formel I können ebenfalls erhalten werden, indem man eine Verbindung der Formel IV mit einer Verbindung der Formel V umsetzt, und anschließend die Schutzgruppen entfernt.

Die Kupplungsreaktion gelingt vorzugsweise in Gegenwart eines Dehydratisierungsmittels, z.B. eines Carbodiimids wie DCCI oder EDCI, ferner z.B. Propanphosphonsäureanhydrid (vgl. Angew. Chem. **1980**, *92*, 129), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, in Dimethylsulfoxid oder in Gegenwart dieser Lösungsmittel, bei Temperaturen zwischen etwa -10 und 40, vorzugsweise zwischen 0 und 30°. Um die intramolekulare Cyclisierung vor der intermolekularen Peptidbindung zu fördern, ist es zweckmäßig, in verdünnten Lösungen zu arbeiten.
Die Reaktionszeit liegt je nach den angewendeten Bedingungen zwischen einigen Minuten und 14 Tagen.

Anstelle von Verbindungen der Formel II und/oder IV können auch Derivate von Verbindungen der Formel II und/oder IV, vorzugsweise eine voraktivierte Carbonsäure, oder ein Carbonsäurehalogenid, ein symmetrisches oder gemischtes Anhydrid oder ein Aktivester eingesetzt werden. Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart) beschrieben.
Aktivierte Ester werden zweckmäßig in situ gebildet, z. B. durch Zusatz von HOBt oder N-Hydroxysuccinimid.

Die Umsetzung erfolgt in der Regel in einem inerten Lösungsmittel, bei Verwendung eines Carbonsäurehalogenids in Gegenwart eines säurebindenden Mittels vorzugsweise einer organischen Base wie Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.

Die Verbindungen der Formel I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die sonst der Formel I entsprechen, aber anstelle einer oder mehrerer freier Amino- und/oder Hydroxygruppen entsprechende geschützte Amino- und/oder Hydroxygruppen enthalten, insbesondere solche, die anstelle einer H-N-Gruppe eine SG¹-N-Gruppe tragen, worin SG¹ eine Aminoschutzgruppe bedeutet und/oder solche, die anstelle des H-Atoms einer Hydroxygruppe eine Hydroxyschutzgruppe tragen, z.B. solche, die der Formel I entsprechen, jedoch anstelle einer Gruppe -COOH eine Gruppe -COOSG² tragen, worin SG² eine Hydroxyschutzgruppe bedeutet.

Es können auch mehrere - gleiche oder verschiedene - geschützte Aminound/oder Hydroxygruppen im Molekül des Ausgangsstoffes vorhanden sein. Falls die vorhandenen Schutzgruppen voneinander verschieden sind, können sie in vielen Fällen selektiv abgespalten werden (vgl. dazu: T.W.

Greene, P.G.M. Wuts, *Protective Groups in Organic Chemistry,* 2. Aufl., Wiley, New York **1991** oder P.J. Kocienski, *Protecting Groups,* 1. Aufl., Georg Thieme Verlag, Stuttgart - New-York, **1994,** H. Kunz, H. Waldmann in *Comprehensive Organic Synthesis,* Vol. 6 (Hrsg. B.M. Trost, I. Fleming, E. Winterfeldt), Pergamon, Oxford, **1991,** S. 631-701).

Der Ausdruck "Aminoschutzgruppe" ist allgemein bekannt und bezieht sich auf Gruppen, die geeignet sind, eine Aminogruppe vor chemischen Umsetzungen zu schützen (zu blockieren). Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20 C-Atomen. Der Ausdruck "Acylgruppe" ist im Zusammenhang mit dem vorliegenden Verfahren im weitesten Sinne aufzufassen. Er umschließt von aliphatischen, araliphatischen, alicyclischen, aromatischen oder heterocyclischen Carbonsäuren oder Sulfonsäuren abgeleitete Acylgruppen sowie insbesondere Alkoxy-carbonyl-, Alkenyloxycarbonyl-, Aryloxycarbonyl- und vor allem Aralkoxy-carbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxy-carbonyl, Boc, 2-lodethoxycarbonyl; Alkenyloxycarbonyl wie Allyloxycarbonyl (Aloc), Aralkyloxycarbonyl wie CBZ (synonym mit Z), 4-Methoxy-benzyloxycarbonyl (MOZ), 4-Nitrobenzyloxycarbonyl oder 9-Fluorenylmethoxycarbonyl (Fmoc); 2-(Phenylsulfonyl)ethoxycarbonyl; Trimethylsilylethoxycarbonyl (Teoc) oder Arylsulfonyl wie 4-Methoxy-2,3,6-trimethylphenyl-sulfonyl (Mtr). Bevorzugte Aminoschutzgruppen sind Boc, Fmoc und Aloc, ferner Z, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen. Typisch für solche Gruppen sind die oben genannten unsubstituierten oder substituierten Aryl-, Aralkyl-, Aroyl- oder Acylgruppen, ferner auch Alkylgruppen, Alkyl-, Aryl- oder Aralkyl-silylgruppen oder O,O- oder O,S-Acetale. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Aralkylgruppen wie Benzyl, 4-Methoxybenzyl oder 2,4-Dimethoxybenzyl, Aroylgruppen wie Benzoyl oder p-Nitrobenzoyl, Acylgruppen wie Acetyl oder Pivaloyl, p-Toluolsulfonyl, Alkylgruppen wie Methyl oder tert.-Butyl, aber auch Allyl, Alkylsilylgruppen wie Trimethylsilyl (TMS), Triisopropylsilyl (TIPS), tert.-Butyldimethylsilyl (TBS) oder Triethylsilyl, Trimethylsilylethyl, Aralkylsilylgruppen wie tert.-Butyldiphenylsilyl (TBDPS), cyclische Acetale wie Isopropyliden-, Cyclopentyliden-, Cyclohexyliden-, Benzyliden-, p-Methoxybenzyliden- oder o,p-Dimethoxybenzylidenacetal, acyclische Acetale wie Tetrahydropyranyl (Thp), Methoxymethyl (MOM), Methoxyethoxymethyl (MEM), Benzyloxymethyl (BOM) oder Methylthiomethyl (MTM). Besonders bevorzugte Hydroxyschutzgruppen sind Benzyl, Acetyl, tert.-Butyl oder TBS.

Das In-Freiheit-Setzen der Verbindungen der Formel I aus ihren funktionellen Derivaten ist für die jeweils benutzte Schutzgruppe aus der Literatur bekannt (z.B. T.W. Greene, P.G.M. Wuts, *Protective Groups in Organic Chemistry*, 2. Aufl., Wiley, New York **1991** oder P.J. Kocienski, *Protecting Groups,* 1. Aufl., Georg Thieme Verlag, Stuttgart - New-York, **1994**). Dabei kann man auch von an sich bekannten, hier nicht näher erwähnten Varianten Gebrauch machen.

Eine Base der Formel I kann mit einer Säure in das zugehörige Säureadditionssalz überführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, schweflige Säure, Dithionsäure, Salpetersäure, Halogenwasserstoffsäuren wie Chlorwasserstoffsäure oder Bromwasserstoffsäure, Phosphorsäuren wie z.B. Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Hexansäure, Octansäure, Decansäure, Hexadecansäure, Octadecansäure, Pivalinsäure, Diethylessigsäure, Malonsäure, Bemsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Benzolsulfonsäure, Trimethoxybenzoesäure, Adamantancarbonsäure, p-Toluolsulfonsäure. Glycolsäure, Embonsäure, Chlorphenoxyessigsäure, Asparaginsäure, Glutaminsäure, Prolin, Glyoxylsäure, Palmitinsäure, Parachlorphenoxyisobuttersäure, Cyclohexancarbonsäure, Glucose-1-phosphat, Naphthalin-mono- und disulfonsäuren oder Laurylschwefelsäure. Salze mit physiologisch nicht unbedenklichen Säuren, z.B. Pikrate, können zur Isolierung und/oder Aufreinigung der Verbindungen der Formel I verwendet werden. Andererseits können Verbindungen der Formel I mit Basen (z.B. Natriumoder Kaliumhydroxid oder -carbonat) in die entsprechenden Metall-, insbesondere Alkalimetall- oder Erdalkalimetall- oder in die entsprechenden Ammoniumsalze umgewandelt werden. Als Salze kommen ferner substituierte Ammoniumsalze, z.B. die Dimethyl-, Diethyloder Diisopropyl-ammoniumsalze, Monoethanol-, Diethanol- oder Diisopropylammoniumsalze, Cyclohexyl-, Dicyclohexylammoniumsalze, Dibenzylethylendiammoniumsalze, weiterhin z.B. Salze mit Arginin oder Lysin.

Gegenstand der Erfindung ist ferner die Verwendung der Verbindungen der Formel I und/oder ihrer physiologisch unbedenklichen Salze zur Herstellung einer pharmazeutischen Zubereitung.

Gegenstand der Erfindung sind ferner pharmazeutische Zubereitungen, enthaltend mindestens eine Verbindung der Formel I und/oder eines ihrer physiologisch unbedenklichen Salze oder Solvate, die insbesondere auf nicht-chemischem Wege hergestellt werden. Hierbei können die Verbindungen der Formel I zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacksund/oder mehrere weitere Wirkstoffe enthalten, z.B. ein oder mehrere Vitamine.
Für die Applikation als Inhalationsspray können Sprays verwendet werden, die den Wirkstoff entweder gelöst oder suspendiert in einem Treibgas oder Treibgasgemisch (z.B. CO₂ oder Fluorchlorkohlenwasserstoffen) enthalten. Zweckmäßig verwendet man den Wirkstoff dabei in mikronisierter Form,
wobei ein oder mehrere zusätzliche physiologisch verträgliche Lösungsmittel zugegen sein können, z.B. Ethanol. Inhalationslösungen können mit Hilfe üblicher Inhalatoren verabreicht werden.

Die Verbindungen der Formel I und ihre physiologisch unbedenklichen Salze können als Integrininhibitoren bei der Bekämpfung von Krankheiten, insbesondere von Thrombosen, Herzinfarkt, koronaren Herzerkrankungen, Arteriosklerose, Tumoren, Osteoporose, Entzündungen und Infektionen verwendet werden.

Die Verbindungen der Formel I nach Anspruch 1 und/oder ihre physiologisch unbedenklichen Salze finden auch Verwendung bei pathologischen Vorgängen, die durch Angiogenese unterhalten oder propagiert werden, insbesondere bei Tumoren oder rheumatoider Arthritis.

Dabei werden die erfindungsgemäßen Substanzen in der Regel in Analogie zu anderen bekannten, im Handel befindlichen Peptiden, insbesondere aber in Analogie zu den in der US-A-4-472 305 beschriebenen Verbindungen verabreicht, vorzugsweise in Dosierungen zwischen etwa 0,05 und 500 mg, insbesondere zwischen 0,5 und 100 mg pro Dosierungseinheit. Die tägliche Dosierung liegt vorzugsweise zwischen etwa 0,01 und 2 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Ferner können die Verbindungen der Formel I als Integrinliganden zur Herstellung von Säulen für die Affinitätschromatographie zur Reindarstellung von integrinen verwendet werden.
Der Ligand, d.h. eine Verbindung der Formel I, wird dabei über eine Ankerfunktion, z.B. die Carboxygruppe von Asp, an einen polymeren Träger kovalent gekuppelt.

Die Herstellung der Materialien für die Affinitätschromatographie zur Integrinreinigung erfolgt unter Bedingungen wie sie für die Kondensation von Aminosäuren üblich und an sich bekannt sind.

Die Verbindungen der Formel I enthalten ein oder mehrere chirale Zentren und können daher in racemischer oder in optisch-aktiver Form vorliegen. Erhaltene Racemate können nach an sich bekannten Methoden mechanisch oder chemisch in die Enantiomeren getrennt werden. Vorzugsweise werden aus dem racemischen Gemisch durch Umsetzung mit einem optisch aktiven Trennmittel Diastereomere gebildet. Als Trennmittel eignen sich z.B. optisch aktive Säuren, wie die D- und L-Formen von Weinsäure, Diacetylweinsäure, Dibenzoylweinsäure, Mandelsäure, Äpfelsäure, Milchsäure oder die verschiedenen optisch aktiven Camphersulfonsäuren wie β-Camphersulfonsäure. Vorteilhaft ist auch eine Enantiomerentrennung mit Hilfe einer mit einem optisch aktiven Trennmittel (z.B. Dinitrobenzoylphenylglycin) gefüllten Säule; als Laufmittel eignet sich z.B. ein Gemisch Hexan/lsopropanol/Acetonitril, z.B. im Volumenverhältnis 82:15:3.

Natürlich ist es auch möglich, optisch aktive Verbindungen der Formel I nach den oben beschriebenen Methoden zu erhalten, indem man Ausgangsstoffe verwendet, die bereits optisch aktiv sind.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

RT = Retentionszeit (Minuten) bei HPLC in den folgenden Systemen: Säulen der Firma Omnicrom YMC:
1. 4,6x250 mm, 5 µm, C₁₈ (Analytik);
2. 30x250 mm, 7µm, C₁₈ (Präparation).
   Als Eluenten kommen Gradienten aus Acetonitril (B) mit 0,1 % TFA und
   Wasser (A) mit 0.1 % TFA zum Einsatz (Angaben jeweils in Volumenprozent Acetonitril). Retentionszeit RT wurde bei einem Fluß von 1 ml/min, ermittelt.
   Detektion bei 220 nm.

Die Trennung der Diastereomeren erfolgt vorzugsweise unter den angegebenen Bedingungen.

Massenspektrometrie (MS): ESI (Elektrospray-lonisation) (M+H)⁺
FAB (Fast-Atom-Bombardment) (M+H)⁺.

### Beispiel 1:

### 1. 5-(9H-Fluoren-9-ylmethoxy)-3H-[1,3,4]oxadiazol-2-on:

Zu einer Lösung von 3,91 mmol Hydrazincarboxylsäure-9H-fluoren-9-ylmethylester in 40 ml Dichlormethan und 40 ml gesättigter wäßriger NaHCO₃-Lösung gibt man 2 Äquivalente Phosgen (1,89M in Toluol; 4,2 ml). Nach 15 Minuten Rühren wird wie üblich aufgearbeitet und man erhält 5-(9H-Fluoren-9-ylmethoxy)-3H-[1,3,4]oxadiazol-2-on ("AB") 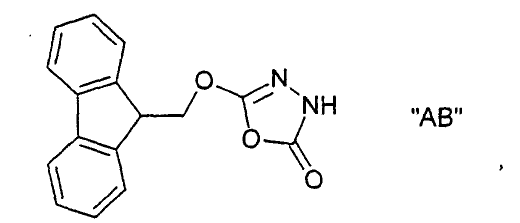 58 mg; IR (KBr): 3300s, 1780s, 1650s, 1451m, 1426m, 1347m, 1224m, 918m, 758w, 740m cm⁻¹.

### 2. Harzgebundenes Fmoc-β-Phe-OH ("BC"):

2,0 g Tritylchloridpolystyrol-Harz (1,8 mmol theoretische Beladung) wird in 20 ml DMF gewaschen. Anschließend wird das Harz mit einer Lösung aus 2,7 mmol Fmoc-β-Phe-OH und 2,25 mmol DIPEA in 20 ml DMF versetzt, 2 Stunden bei Raumtemperatur geschüttelt und danach wird 1 ml Methanol zugegeben. Man wäscht mit DCM (5 x 20 ml) und Methanol (3 x 20 ml) und trocknet.

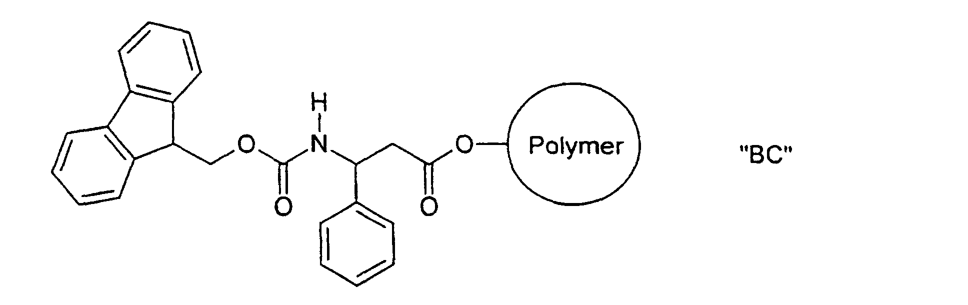

### 3. Harzgebundenes Fmoc-azaGly-β-Phe ("CD"):

0,48 mmol/g "BC" wird mit DCM (2 x 7 ml) und mit NMP (1 x 7 ml) gewaschen und anschließend zweimal mit 20% Piperidin in 7 ml DMF zuerst 5 min. und danach 15 min. entschützt. Das entschützte Harz wird mit NMP (5 x 7 ml) und absolutem DCM (5 x 7 ml) gewaschen und mit einer Lösung aus 0,72 mmol "AB" in 7 ml DCM versetzt und 90 min. bei Raumtemperatur geschüttelt.
Man wäscht mit DCM (5 x 7 ml) und NMP (5 x 7 ml) und trocknet das Harz.

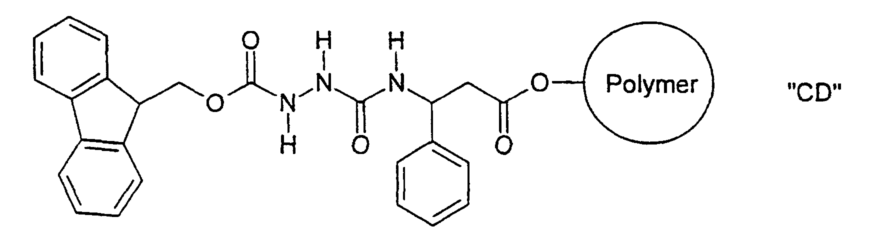

### 4. 3-[4-(3-Guanidinobenzoyl)-semicarbazido]-3-phenylpropionsäure

0,24 mmol "CD" wird mit NMP (7 x 5 ml) gewaschen und zweimal mit 20% Piperidin in 7 ml DMF zunächst 5 min. und danach 15 min. entschützt. Anschließend wird das Harz mit NMP (5 x 7 ml) und DMF (2 x 7 ml) gewaschen und mit einer Lösung aus 0,48 mmol 3-(9H-Fluoren-9-ylmethoxycarbonylamino)-benzoesäure, 0,48 mmol HATU und 2,4 mmol Collidin in 5 ml DMF versetzt und 90 min. bei Raumtemperatur geschüttelt. Man wäscht und entschützt wie beschrieben.

Das Harz wird anschließend mit einer Lösung aus 2,4 mmol N,N'-bis-BOC-1-guanylpyrazol ("DE") in 4 ml Chloroform versetzt und 16 Stunden bei 50° belassen. Man wäscht mit DCM, Methanol und Diethylether. Zur Abspaltung der BOC-Gruppen wird das Harz mit einem Gemisch aus 95% TFA und 5% Triisopropylsilan (5 ml) bei Raumtemperatur zunächst 90 min. und danach 30 min. geschüttelt. Entfernen des Lösungsmittels und Reinigung mit präparativer RP-HPLC ergibt, 3-[4-(3-Guanidinobenzoyl)-semicarbazido]-3-phenylpropionsäure, Trifluoracetat.
RT = 19,1 (0→50% B, 30 min.)
MS (ESI): m/z = 385.1 ([M+H]⁺).

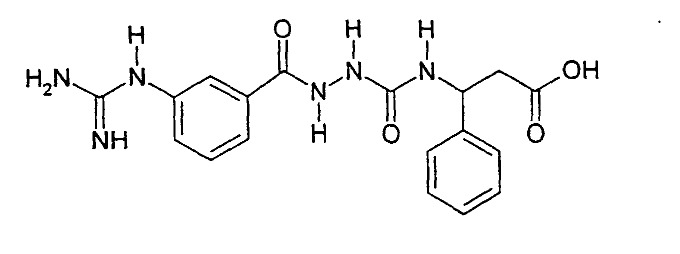

### Beispiel 2:

### 1. Analog zu Beispiel 1 erhält man durch Umsetzung von "CD"

mit 3-(9H-Fluoren-9-ylmethoxycarbonylamino)-phenylessigsäure, anschließender Abspaltung der Fmoc-Gruppe und Reaktion mit "DE" sowie Abspaltung der BOC-Gruppe und vom Harz
3-[4-(3-Guanidinophenylacetyl)-semicarbazido]-3-phenylpropionsäure, Trifluoracetat.
RT = 4,3 (10→50% B, 30 min.)
MS (ESI): m/z = 399.1 ([M+H]⁺).

### 2. Analog zu Beispiel 1 erhält man durch Umsetzung von "EF", hergestellt durch Umsetzung von "AB" mit harzgebundenem Fmoc-geschützten β-(4-Chlorphenyl)-alanin

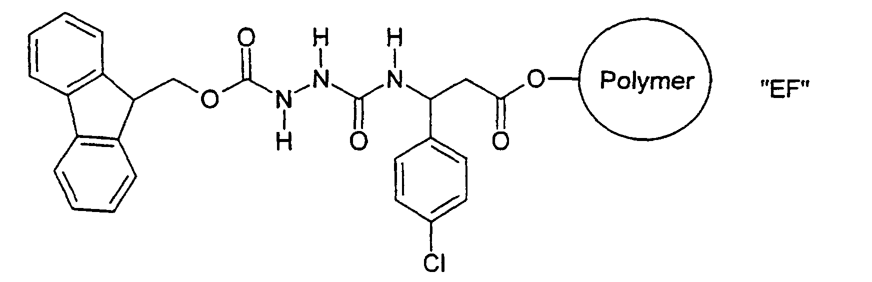

mit 3-(9H-Fluoren-9-ylmethoxycarbonylamino)-benzoesäure, anschließender Abspaltung der Fmoc-Gruppe und Reaktion mit "DE" sowie Abspaltung der BOC-Gruppe und vom Harz
3-[4-(3-Guanidinobenzoyl)-semicarbazido]-3-(4-chlorphenyl)-propionsäure, Trifluoracetat.
RT = 22,8 (0→50% β, 30 min.)
MS (ESI): m/z = 419.0 ([M+H]⁺).

### Beispiel 3:

0,24 mmol "CD" wird mit NMP (7 x 5 ml) gewaschen und zweimal mit 20% Piperidin in 7 ml DMF zunächst 5 min. und danach 15 min. entschützt. Anschließend wird das Harz mit NMP (5 x 7 ml) und DMF (2 x 7 ml) gewaschen und mit einer Lösung aus 0,48 mmol 3-(9H-Fluoren-9-ylmethoxycarbonylamino)-valeriansäure, 0,48 mmol HATU und 2,4 mmol Collidin in 5 ml DMF versetzt und 90 min. bei Raumtemperatur geschüttelt. Man wäscht und entschützt wie beschrieben.
Das Harz wird anschließend mit einer Lösung aus 2,4 mmol N,N'-bis-BOC-1-guanylpyrazol ("DE") in 4 ml Chloroform versetzt und 16 Stunden bei 50° belassen. Man wäscht mit DCM. Zur Abspaltung der BOC-Gruppen wird das Harz mit einem Gemisch aus 95% TFA und 5% Triisopropylsilan (5 ml) bei Raumtemperatur zunächst 90 min. und danach 30 min. geschüttelt. Entfernen des Lösungsmittels und Reingung mit präparativer RP-HPLC ergibt, 3-[4-(3-Guanidinopentanoyl)-semicarbazido]-3-phenylpropionsäure, Trifluoracetat.
RT = 18,2 (0→50% B, 30 min.)
MS (ESI): m/z = 365.1 ([M+H]⁺).

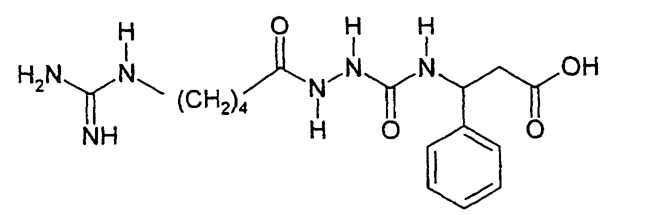

### Beispiel 4:

Analog zu Beispiel 3 erhält man durch Umsetzung von "EF"
mit 3-(9H-Fluoren-9-ylmethoxycarbonylamino)-valeriansäure, anschließender Abspaltung der Fmoc-Gruppe und Reaktion mit "DE" sowie Abspaltung der BOC-Gruppe und vom Harz
3-[4-(3-Guanidinopentanoyl)-semicarbazido]-3-(4-chlorphenyl)-propionsäure, Trifluoracetat.
RT = 21,9 (0→50% B, 30 min.)
MS (ESI): m/z = 399.1 ([M+H]⁺).

### Beispiel 5:

0,60 mmol "EF" wird mit 20 ml NMP gewaschen und zweimal mit 20% Piperidin in 20 ml DMF zunächst 5 min. und danach 15 min. entschützt. Anschließend wird das Harz mit NMP (5 x 20 ml) und DMF (2 x 20 ml) gewaschen und mit einer Lösung aus 0,90 mmol N-(2-(4-Methylpyridyl)-5-aminovaleriansäure, 0,90. mmol HATU und 6,0 mmol Collidin in 5 ml DMF versetzt und über Nacht bei Raumtemperatur geschüttelt. Man wäscht mit DMF, NMP und DCM. Zur Abspaltung von der festen Phase schüttelt man das gewaschene Harz mit 20 ml eines Gemisches aus DCM/Essigsäure/Trifluorethanol (3:1:1) bei Raumtemperatur zunächst 90 min. und danach 30 min.
Entfernen des Lösungsmittels ergibt 3-{4-[5-(4-Methyl-pyridin-2-ylamino)-pentanoyl]-semicarbazido}-3-(4-chlorphenyl)-propionsäure, Acetat.
Die Reinigung mit präparativer HPLC ergibt 3-{4-[5-(4-Methyl-pyridin-2-ylamino)-pentanoyl]-semicarbazido}-3-(4-chlorphenyl)-propionsäure, Trifluoracetat.
RT = 19,3 (10→60% B, 30 min.)
MS (ESI): m/z = 448.1 ([M+H]⁺).

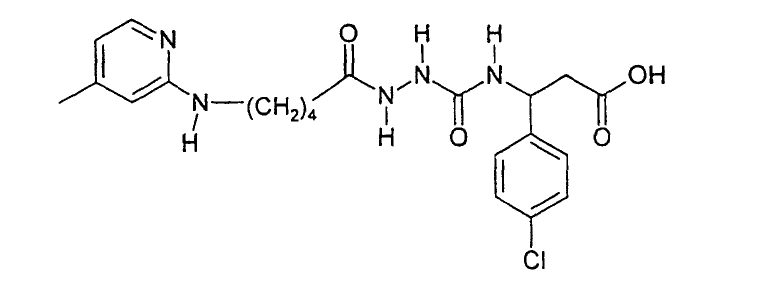

### Beispiel 6:

### 1. Analog zu Beispiel 5 erhält man durch Umsetzung von "FG", hergestellt durch Umsetzung von "AB" mit harzgebundenem Fmoc-geschützten β-(3,5-Dichlorphenyl)-alanin,

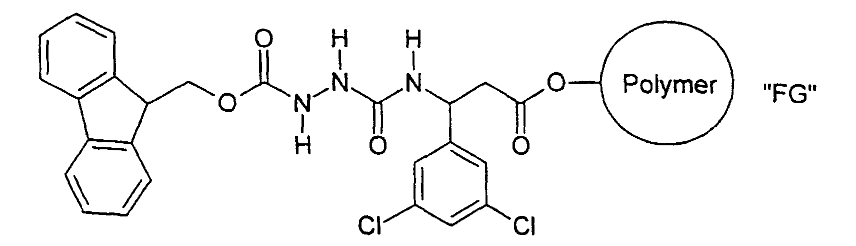

mit N-(2-(4-Methylpyridyl)5-aminovaleriansäure sowie Abspaltung vom Harz

3-{4-[5-(4-Methyl-pyridin-2-ylamino)-pentanoyl]-semicarbazido}-3-(3,5-dichlorphenyl)-propionsäure, Acetat.
Die Reinigung mittels präparativer HPLC ergibt 3-{4-[5-(4-Methyl-pyridin-2-ylamino)-pentanoyl]-semicarbazido}-3-(3,5-dichlorphenyl)-propionsäure, Trifluoracetat.
RT = 19,3 (0→60% B, 30 min.)
MS (ESI): m/z = 483.4 ([M+H]⁺).
Wird die Reaktionslösung nach der Abspaltung vom Harz und nach Abfiltrieren der festen Phase mit Salzsäure auf einen pH-Wert von 4.0 angesäuert, so entsteht dabei das innere Salz der
3-{4-[5-(4-Methyl-pyridin-2-ylamino)-pentanoyl]-semicarbazido}-3-(3,5-dichlorphenyl)-propionsäure.
RT = 19,2 (0→60% B, 30 min.)

### 2. Analog zu Beispiel 5 erhält man durch Umsetzung von "GH", hergestellt durch Umsetzung von "AB" mit harzgebundenem Fmoc-geschützten β-(3-Nitrophenyl)-alanin,

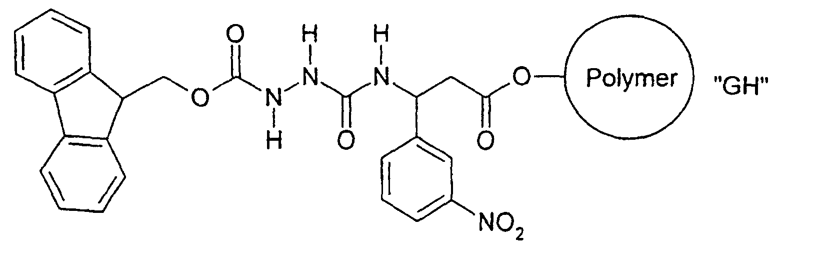

mit N-(2-(4-Methylpyridyl)5-aminovaleriansäure sowie Abspaltung vom Harz
3-{4-[5-(4-Methyl-pyridin-2-ylamino)-pentanoyl]-semicarbazido}-3-(3-nitrophenyl)-propionsäure, Acetat.
Nach Reinigung mittels präparativer HPLC erhält man 3-{4-[5-(4-Methylpyridin-2-ylamino)-pentanoyl]-semicarbazido}-3-(3-nitrophenyl)-propionsäure, Trifluoracetat.
RT = 14,9 (0→60% B, 30 min.)
MS (ESI): m/z = 459.5 ([M+H]⁺).

### 3. Analog zu Beispiel 5 erhält man durch Umsetzung von "HK", hergestellt durch Umsetzung von "AB" mit harzgebundenem Fmoc-geschützten β-(4-Fluorphenyl)-phenylalanin,

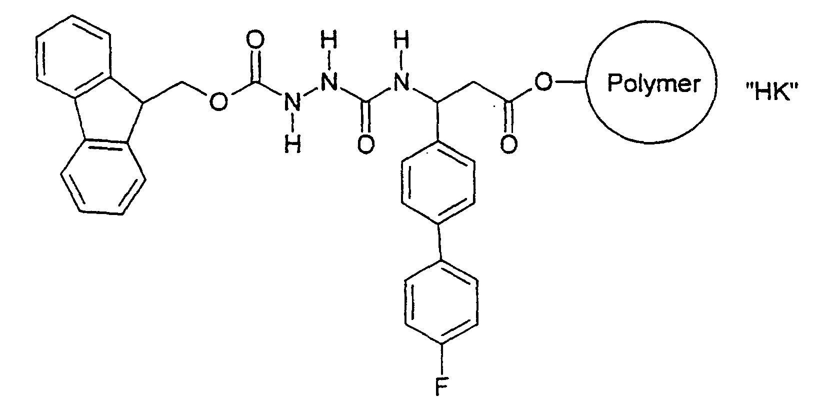

mit N-(2-(4-Methylpyridyl)5-aminovaleriansäure sowie Abspaltung vom Harz
3-{4-[5-(4-Methyl-pyridin-2-ylamino)-pentanoyl]-semicarbazido}-3-(4'-(4-Fluor)-biphenyl)-propionsäure, Acetat.
Nach Aufreinigung mittels präparativer HPLC erhält man 3-{4-[5-(4-Methylpyridin-2-ylamino)-pentanoyl]-semicarbazido}-3-(4'-(4-Fluor)-biphenyl)-propionsäure, Trifluoracetat.

### 4. Analog zu Beispiel 5 erhält man durch Umsetzung von "KL", hergestellt durch Umsetzung von "AB" mit harzgebundenem Fmoc-geschützten β-(2-Nitrophenyl)-alanin,

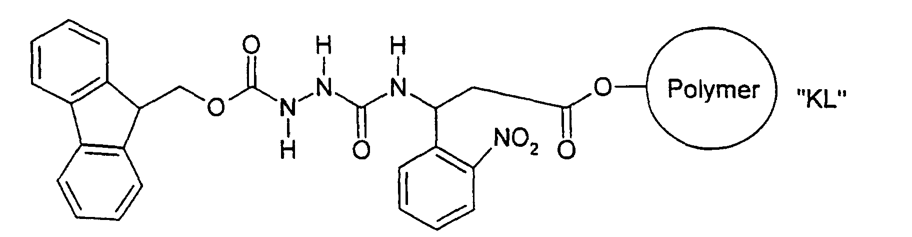

mit N-(2-(4-Methylpyridyl)5-aminovaleriansäure sowie Abspaltung vom Harz
3-{4-[5-(4-Methyl-pyridin-2-ylamino)-pentanoyl]-semicarbazido}-3-(2-nitrophenyl)-propionsäure, Acetat.
Nach Aufreinigung mittels präparativer HPLC erhält man 3-{4-[5-(4-Methylpyridin-2-ylamino)-pentanoyl]-semicarbazido}-3-(2-nitrophenyl)-propionsäure, Trifluoracetat.
RT = 14,7 (0→60% B, 30 min.)
MS (ESI): m/z = 459.5 ([M+H]⁺).

### 5. Analog zu Beispiel 5 erhält man durch Umsetzung von "LM", hergestellt durch Umsetzung von "AB" mit harzgebundenem Fmoc-geschützten β-(4-Trifluormethoxyphenyl)-alanin,

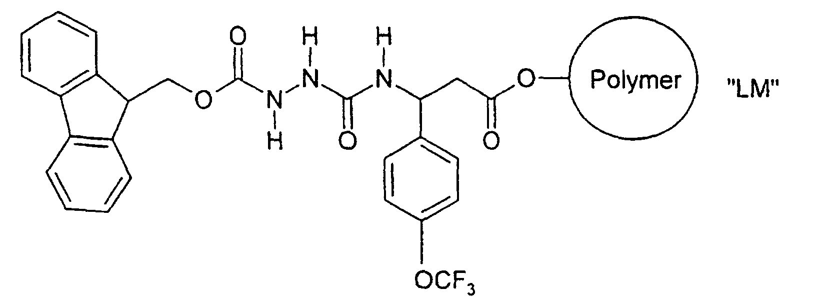

mit N-(2-(4-Methylpyridyl)5-aminovaleriansäure sowie Abspaltung vom Harz
3-{4-[5-(4-Methyl-pyridin-2-ylamino)-pentanoyl]-semicarbazido}-3-(4-trifluormethoxyphenyl)-propionsäure, Acetat.
Nach Aufreinigung mittels präparativer HPLC erhält man 3-{4-[5-(4-Methylpyridin-2-ylamino)-pentanoyl]-semicarbazido}-3-(4-trifluormethoxyphenyl)-propionsäure, Trifluoracetat.
RT = 19,8 (0→60% B, 30 min.)
MS (ESI): m/z = 498.5 ([M+H]⁺).

### Beispiel 7:

Analog zu Beispiel 1.2 erhält man durch Umsetzung von Tritylchloridpolystyrol-Harz mit Fmoc-geschützter 3-Aminopropionsäure das Harz "LO" 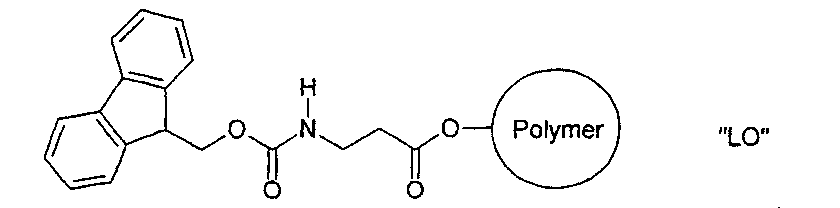 welches analog zu Beispiel 1.3 entschützt und mit "AB" zu "MN" 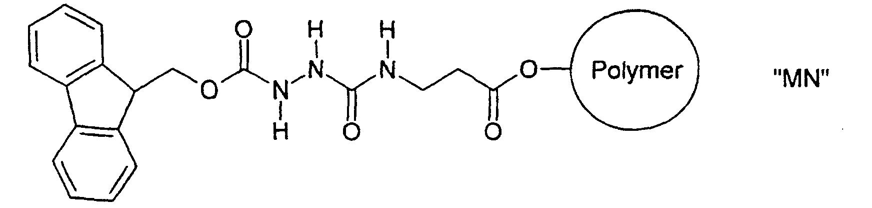 umgesetzt wird.

Analog zu Beispiel 5 wird "MN" mit N-(2-(4-Methylpyridyl)-5-aminovaleriansäure umgesetzt. Man erhält nach Abspaltung vom Harz 3-{4-[5-(4-Methyl-pyridin-2-ylamino)-pentanoyl]-semicarbazido}-3-propionsäure, Acetat.

Nach Aufreinigung mittels präparativer HPLC erhält man 3-{4-[5-(4-Methylpyridin-2-ylamino)-pentanoyl]-semicarbazido}-3-propionsäure, Trifluoracetat.
RT = 7,3 (0→60% B, 30 min.)
MS (ESI): m/z = 338.4 ([M+H]⁺).

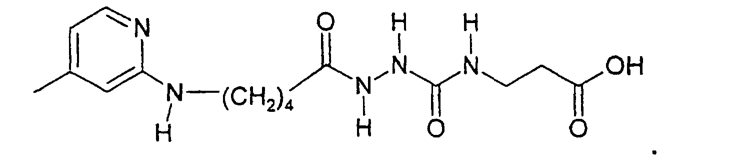

### Beispiel 8:

### 1. 3-Phenylglutarsäureanhydrid

10 mmol 3-Phenylglutarsäure und 30 mmol Essigsäureanhydrid werden bis zur vollständigen Lösung unter Rückfluß erhitzt. Nach dem Abkühlen werden 3 ml Diethylether zugegeben, der Niederschlag abfiltriert und mit Diethylether gewaschen. Man erhält 3-Phenylglutarsäureanhydrid.

¹H-NMR (250 MHz, CDCl₃): δ = 2,85 (dd, 2H, CH(*H*CH)₂), 3,1 (dd, 2H, CH(HC*H*)₂), 3,4 (m, 1H, C*H*(CH₂)₂), 7,15-7,45 (m, 5H, arom. H).

### 2. 5-[N'-(9H-Fluoren-9-ylmethoxycarbonyl)-hydrazino]-5-oxo-3-phenylpentansäure

2,0 mmol 3-Phenylglutarsäureanhydrid und 2,0 mmol Fmoc-Hydrazin werden in 30 ml THF gelöst und 16 Stunden unter Rückfluß erhitzt. Anschließend wird das Produkt mit 50 ml DCM und 50 ml 1N HCI-Lösung extrahiert, die organische Phase mit MgSO₄ getrocknet und abfiltriert. Man erhält nach entfernen des Lösungsmittels 5-[N'-(9H-Fluoren-9-ylmethoxycarbonyl)-hydrazino]-5-oxo-3-phenyl-pentansäure.

RT = 10.4 (30→80 % B, 30 min); MS (ESI): m/z = 910.8 ([2M+Na]⁺).

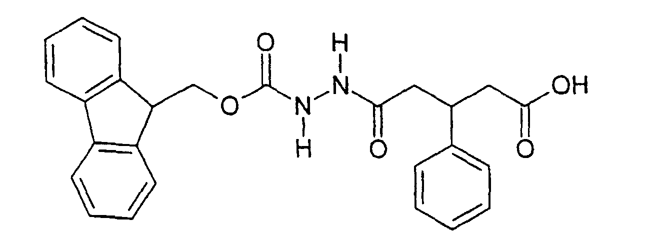

### 3. 5-[N'-(3-Guanidino-benzoyl)-hydrazino]-5-oxo-3-phenyl-pentansäure

5-[N'-(9H-Fluoren-9-ylmethoxycarbonyl)-hydrazino]-5-oxo-3-phenylpentansäure wird analog wie in Beispiel 1 beschrieben entschützt. Anschließend wird die entschützte Verbindung mit einer Lösung aus 2,0 mmol N,N'-bis-BOC-1-guanylpyrazol ("DE") in 4 ml Chloroform versetzt und 16 Stunden bei 50° belassen. Man entfernt das Lösungsmittel im Vakuum. Zur Abspaltung der BOC-Gruppen wird mit einem Gemisch aus 95% TFA und 5% Triisopropylsilan (5 ml) bei Raumtemperatur zunächst 90 min. und danach 30 min. gerührt. Entfernen des Lösungsmittels und Reinigung mit präparativer RP-HPLC ergibt 5-[N'-(3-Guanidino-benzoyl)-hydrazino]-5-oxo-3-phenyl-pentansäure, Acetat.
RT = 17,0 (10→50% B, 30 min.)
MS (ESI): m/z = 384.1 ([M+H]⁺).

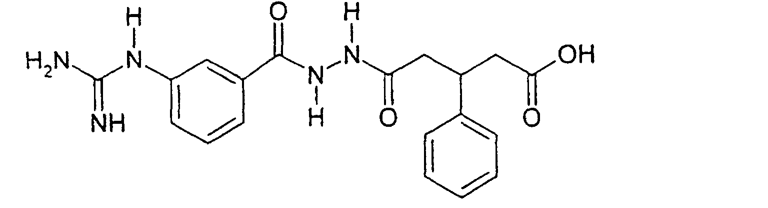

### Beispiel 9:

### 1. Analog zu Beispiel 5 erhält man durch Umsetzung von 5-[N'-(9H-Fluoren-9-ylmethoxycarbonyl)-hydrazino]-5-oxo-3-phenyl-pentansäure

mit N-(2-(4-Methylpyridyl)5-aminovaleriansäure
4-{N'-[5-(4-Methyl-pyridin-2-ylamino)-pentanoyl]-hydrazinocarbonyl}-3-phenyl-buttersäure, Acetat.
RT = 22,1 (10→60% B, 30 min.)
MS (ESI): m/z = 413,1 ([M+H]⁺).

### 2. Analog zu Beispiel 5 erhält man durch Umsetzung von (3R)-5-[N'-(9H-Fluoren-9-ylmethoxycarbonyl)-hydrazino]-5-oxo-3-(4-chlorphenyl)-pentansäure

mit N-(2-(4-Methylpyridyl)5-aminovaleriansäure
(3R)-4-(N'-[5-(4-Methyl-pyridin-2-ylamino)-pentanoyl]-hydrazinocarbonyl}-3-(4-chlorphenyl)-buttersäure, Acetat.
MS (FAB): m/z = 447.9 ([M+H]⁺).
Wird die Reaktionslösung nach der Abspaltung vom Harz und nach Abfiltrieren der festen Phase mit Salzsäure auf einen pH-Wert von 4.0 angesäuert, so entsteht dabei das innere Salz der
(3R)-4-{N'-[5-(4-Methyl-pyridin-2-ylamino)-pentanoyl]-hydrazinocarbonyl}-3-(4-chlorphenyl)-buttersäure.

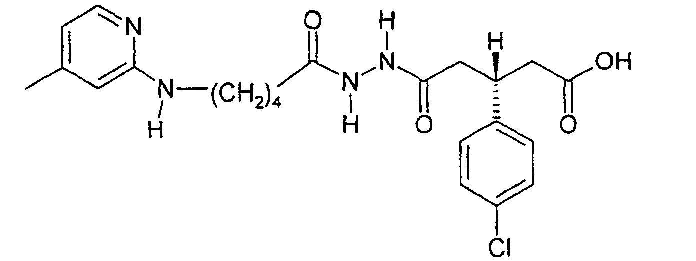

### Beispiel 10:

Analog zu Beispiel 3 erhält man durch Umsetzung von 5-[N'-(9H-Fluoren-9-ylmethoxycarbonyl)-hydrazino]-5-oxo-3-(4-chlorphenyl)-pentansäure mit 3-(9H-Fluoren-9-ylmethoxycarbonylamino)-valeriansäure, anschließender Abspaltung der Fmoc-Gruppe und Reaktion mit "DE" sowie Abspaltung der BOC-Gruppe
4-[N'-(5-Guanidino-pentanoyl)-hydrazinocarbonyl]-3-phenylbuttersäure, Trifluoracetat.
RT = 19,4 (0→50% B, 30 min.)
MS (ESI): m/z = 364.2 ([M+H]⁺).

### Beispiel 11:

Analog zu Beispiel 5 erhält man durch Umsetzung von 5-[N'-(9H-Fluoren-9-ylmethoxycarbonyl)-hydrazino]-5-oxo-pentansäure, hergestellt analog zu Beispiel 8.2 aus der Umsetzung von Glutarsäureanhydrid mit Fmoc-Hydrazin,
mit N-(2-(4-Methylpyridyl)-5-aminovaleriansäure
4-{N'-[5-(4-Methyl-pyridin-2-ylamino)-pentanoyl]-hydrazinocarbonyl}buttersäure, Acetat.
MS (ESI): m/z = 337,4 ([M+H]⁺).

### Beispiel 12:

1. Analog zu Beispiel 5 erhält man durch Umsetzung von "NO", hergestellt durch Umsetzung von "AB" mit harzgebundenem Fmoc-geschützten β-(4-Bromphenyl)-alanin, 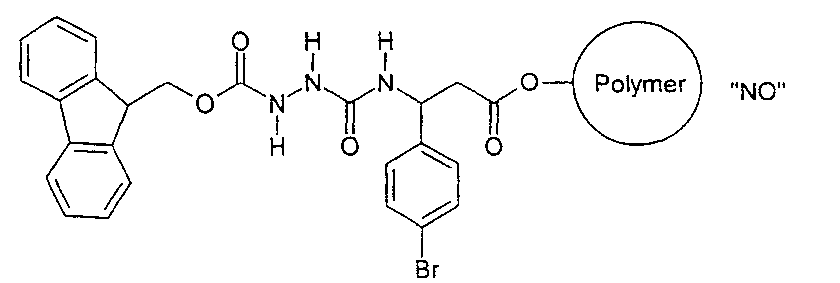 mit N-(2-(4-Methylpyridyl)5-aminovaleriansäure sowie Abspaltung vom Harz
   3-{4-[5-(4-Methyl-pyridin-2-ylamino)-pentanoyl]-semicarbazido}-3-(4-bromphenyl)-propionsäure, Acetat.
   Nach präparativer HPLC erhält man 3-{4-[5-(4-Methyl-pyridin-2-ylamino)-pentanoyl]-semicarbazido}-3-(4-bromphenyl)-propionsäure, Trifluoracetat. RT = 17,0 (0→60% B, 30 min.)
   MS (ESI): m/z = 493.4 ([M+H]⁺).
2. Analog zu Beispiel 5 erhält man durch Umsetzung von "OP", hergestellt durch Umsetzung von "AB" mit harzgebundenem Fmoc-geschützten β-(Naphthalin-1-yl)-alanin, 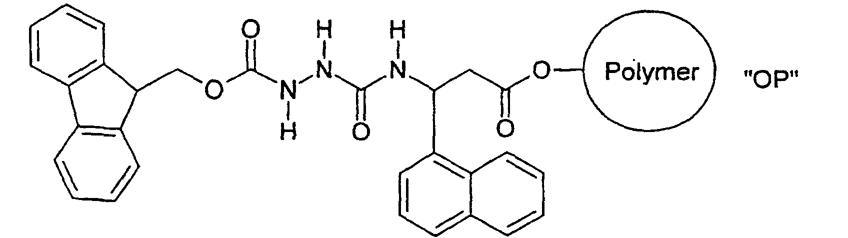 mit N-(2-(4-Methylpyridyl)5-aminovaleriansäure sowie Abspaltung vom Harz
   3-{4-[5-(4-Methyl-pyridin-2-ylamino)-pentanoyl]-semicarbazido}-3-(naphthalin-1-yl)-propionsäure, Acetat.
   Nach präparativer HPLC erhält man 3-{4-[5-(4-Methyl-pyridin-2-ylamino)-pentanoyl]-semicarbazido}-3-(naphthalin-1-yl)-propionsäure, Trifluoracetat. RT = 17,6 (0→60% B, 30 min.)
   MS (ESI): m/z = 464.5 ([M+H]⁺).

### Beispiel 13:

1. Analog zu Beispiel 1 erhält man durch Umsetzung von "LM", hergestellt durch Umsetzung von "AB" mit harzgebundenem Fmoc-geschützten β-(4-Trifluormethoxyphenyl)-alanin, 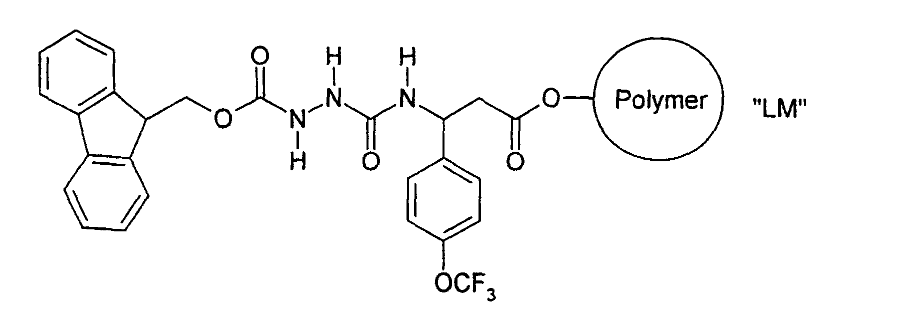 mit 3-(9H-Fluoren-9-ylmethoxycarbonylamino)-benzoesäure,
   anschließender Abspaltung der Fmoc-Gruppe und Reaktion mit "DE" sowie Abspaltung der BOC-Gruppe und vom Harz
   3-[4-(3-Guanidinobenzoyl)-semicarbazido]-3-(4-trifluormethoxyphenyl)-propionsäure, Trifluoracetat.
   RT = 17,6 (0→60% B, 30 min.)
   MS (ESI): m/z = 468.5 ([M+H]⁺).

### Beispiel 14:

1. Analog zu Beispiel 1 erhält man durch Umsetzung von "FG" mit 3-(9H-Fluoren-9-ylmethoxycarbonylamino)-benzoesäure, anschließender Abspaltung der Fmoc-Gruppe und Reaktion mit "DE" sowie Abspaltung der BOC-Gruppe und vom Harz
   3-[4-(3-Guanidinobenzoyl)-semicarbazido]-3-(3,5-dichlorphenyl)-propionsäure, Trifluoracetat.
   RT = 17,0 (0→60% B, 30 min.)
   MS (ESI): m/z = 454.3 ([M+H]⁺).
2. Analog zu Beispiel 1 erhält man durch Umsetzung von "OP" mit 3-(9H-Fluoren-9-ylmethoxycarbonylamino)-benzoesäure, anschließender Abspaltung der Fmoc-Gruppe und Reaktion mit "DE" sowie Abspaltung der BOC-Gruppe und vom Harz
   3-[4-(3-Guanidinobenzoyl)-semicarbazido]-3-naphthyl-propionsäure, Trifluoracetat.
   RT = 15,9 (0→60% B, 30 min.)
   MS (ESI): m/z = 435.5 ([M+H]⁺).
3. Analog zu Beispiel 1 erhält man durch Umsetzung von "NO" mit 3-(9H-Fluoren-9-ylmethoxycarbonylamino)-benzoesäure, anschließender Abspaltung der Fmoc-Gruppe und Reaktion mit "DE" sowie Abspaltung der BOC-Gruppe und vom Harz
   3-[4-(3-Guanidinobenzoyl)-semicarbazido]-3-(4-bromphenyl)-propionsäure, Trifluoracetat.
   RT = 14,9 (0→60% B, 30 min.)
   MS (ESI): m/z = 464.3 ([M+H]⁺).
4. Analog zu Beispiel 5 erhält man durch Umsetzung von "CD" mit N-(2-(4-Methylpyridyl)5-aminovaleriansäure sowie Abspaltung vom Harz
   3-{4-[5-(4-Methyl-pyridin-2-ylamino)-pentanoyl]-semicarbazido}-3-phenyl-propionsäure, Acetat.
   Nach präparativer HPLC erhält man 3-{4-[5-(4-Methyl-pyridin-2-ylamino)-pentanoyl]-semicarbazido}-3-phenyl-propionsäure, Trifluoracetat.
   RT = 13,2 (0→60% B, 30 min.)
   MS (ESI): m/z = 414.5 ([M+H]⁺).

### Beispiel 15:

1. Analog zu Beispiel 9 erhält man durch Umsetzung von 5-[N'-(9H-Fluoren-9-ylmethoxycarbonyl)-hydrazino]-5-oxo-3-(4-bromphenyl)-pentansäure, hergestellt durch Umsetzung von 3-(4-Bromphenyl)-glutarsäureanhydrid mit Fmoc-Hydrazin,
   mit N-(2-(4-Methylpyridyl)5-aminovaleriansäure
   3-(4-Bromphenyl)-4-{N'-[5-(4-methyl-pyridin-2-ylamino)-pentanoyl]-hydrazinocarbonyl}-buttersäure, Acetat.
   Nach präparativer HPLC erhält man 3-(4-Bromphenyl)-4-{N'-[5-(4-methylpyridin-2-ylamino)-pentanoyl]-hydrazinocarbonyl}-buttersäure, Trifluoracetat.
   RT = 17,0 (0→60% B, 30 min.)
   MS (ESI): m/z = 492.4 ([M+H]⁺).
2. Analog zu Beispiel 8 erhält man durch Umsetzung von 5-[N'-(9H-Fluoren-9-ylmethoxycarbonyl)-hydrazino]-5-oxo-3-(4-bromphenyl)-pentansäure mit "DE"
   3-(4-Bromphenyl)-5-{N'-[1-(3-guanidinophenyl)-methanoyl]-hydrazino}-5-oxo-pentansäure, Trifluoracetat.
   RT = 15,0 (0→60% B, 30 min.)
   MS (ESI): m/z = 463.3 ([M+H]⁺).
3. Analog zu Beispiel 1 erhält man durch Umsetzung von 5-[N'-(9H-Fluoren-9-ylmethoxycarbonyl)-hydrazino]-5-oxo-3-(4-bromphenyl)-pentansäure mit 3-(9H-Fluoren-9-ylmethoxycarbonylamino)-benzoesäure und Thioacetimidsäure-naphthalin-2-ylmethylester
   5-{N'-[1-(3-Acetimidoylamino-phenyl)-methanoyl]-hydrazino}-3(4-bromphenyl)-5-oxo-pentansäure, Trifluoracetat.
   RT = 15,4 (0→60% B, 30 min.)
   MS (ESI): m/z = 462.3 ([M+H]⁺).

### Beispiel 16:

1. Analog zu Beispiel 9 erhält man durch Umsetzung von 5-[N'-(9H-Fluoren-9-ylmethoxycarbonyl)-hydrazino]-5-oxo-3-(4-fluorphenyl)-pentansäure, hergestellt durch Umsetzung von 3-(4-Fluorphenyl)-glutarsäureanhydrid mit Fmoc-Hydrazin,
   mit N-(2-(4-Methylpyridyl)5-aminovaleriansäure
   3-(4-Fluorphenyl)-4-{N'-[5-(4-methyl-pyridin-2-ylamino)-pentanoyl]-hydrazinocarbonyl}-buttersäure, Acetat.
   Nach präparativer HPLC erhält man 3-(4-Fluorphenyl)-4-(N'-[5-(4-methylpyridin-2-ylamino)-pentanoyl]-hydrazinocarbonyl}-buttersäure, Trifluoracetat.
   RT = 14,1 (0→60% B, 30 min.)
   MS (ESI): m/z = 431.5 ([M+H]⁺).
2. Analog zu Beispiel 8 erhält man durch Umsetzung von 5-[N'-(9H-Fluoren-9-ylmethoxycarbonyl)-hydrazino]-5-oxo-3-(4-fluorphenyl)-pentansäure mit "DE"
   3-(4-Fluorphenyl)-5-{N'-[1-(3-guanidinophenyl)-methanoyl]-hydrazino}-5-oxo-pentansäure, Trifluoracetat.
   RT = 11,2 (0→60% B, 30 min.)
   MS (ESI): m/z = 402.4 ([M+H]⁺).
3. Analog zu Beispiel 1 erhält man durch Umsetzung von 5-[N'-(9H-Fluoren-9-ylmethoxycarbonyl)-hydrazino]-5-oxo-3-(4-fluorphenyl)-pentansäure mit 3-(9H-Fluoren-9-ylmethoxycarbonylamino)-benzoesäure und Thioacetimidsäure-naphthalin-2-ylmethylester
   5-{N'-[1-(3-Acetimidoylamino-phenyl)-methanoyl]-hydrazino}-3(4-fluorphenyl)-5-oxo-pentansäure, Trifluoracetat.
   RT = 12,0 (0→60% B, 30 min.)
   MS (ESI): m/z = 401.4 ([M+H]⁺).

### Beispiel 17:

1. Analog zu Beispiel 9 erhält man durch Umsetzung von 5-[N'-(9H-Fluoren-9-ylmethoxycarbonyl)-hydrazino]-5-oxo-3-(4-chlorphenyl)-pentansäure, hergestellt durch Umsetzung von 3-(4-Chlorphenyl)-glutarsäureanhydrid mit Fmoc-Hydrazin,
   mit N-(2-(4-Methylpyridyl)5-aminovaleriansäure
   3-(4-Chlorphenyl)-4-{N'-[5-(4-methyl-pyridin-2-ylamino)-pentanoyl]-hydrazinocarbonyl}-buttersäure, Acetat.
   Nach präparativer HPLC erhält man 3-(4-Chlorphenyl)-4-{N'-[5-(4-methylpyridin-2-ylamino)-pentanoyl]-hydrazinocarbonyl}-buttersäure,
   Trifluoracetat.
   RT = 16,3 (0→60% B, 30 min.)
   MS (ESI): m/z = 447.9 ([M+H]⁺).
2. Analog zu Beispiel 8 erhält man durch Umsetzung von 5-[N'-(9H-Fluoren-9-ylmethoxycarbonyl)-hydrazino]-5-oxo-3-(4-chlorphenyl)-pentansäure mit "DE"
   3-(4-Chlorphenyl)-5-{N'-[1-(3-guanidinophenyl)-methanoyl]-hydrazino}-5-oxo-pentansäure, Trifluoracetat.
   RT = 13,9 (0→60% B, 30 min.)
   MS (ESI): m/z = 418.9 ([M+H]⁺).
3. Analog zu Beispiel 1 erhält man durch Umsetzung von 5-[N'-(9H-Fluoren-9-ylmethoxycarbonyl)-hydrazino]-5-oxo-3-(4-chlorphenyl)-pentansäure mit 3-(9H-Fluoren-9-ylmethoxycarbonylamino)-benzoesäure und Thioacetimidsäure-naphthalin-2-ylmethylester
   5-{N'-[1-(3-Acetimidoylamino-phenyl)-methanoyl]-hydrazino}-3-(4-chlorphenyl)-5-oxo-pentansäure, Trifluoracetat.
   MS (ESI): m/z = 417.9 ([M+H]⁺).
4. Analog zu Beispiel 3 erhält man durch Umsetzung von 5-[N'-(9H-Fluoren-9-ylmethoxycarbonyl)-hydrazino]-5-oxo-3-(4-chlorphenyl)-pentansäure mit 3-(9H-Fluoren-9-ylmethoxycarbonylamino)-valeriansäure und "DE"
   3-(4-Chlorphenyl)-5-[N'-(5-guanidino-pentanoyl)-hydrazino]-5-oxopentansäure, Trifluoracetat.
   RT = 13,7 (0→60% B, 30 min.)
   MS (ESI): m/z = 398.5 ([M+H]⁺).

### Beispiel 18:

1. Analog zu Beispiel 9 erhält man durch Umsetzung von 5-[N'-(9H-Fluoren-9-ylmethoxycarbonyl)-hydrazino]-5-oxo-3-(3,5-dichlorphenyl)-pentansäure, hergestellt durch Umsetzung von 3-(3,5-Dichlorphenyl)-glutarsäureanhydrid mit Fmoc-Hydrazin,
   mit N-(2-(4-Methylpyridyl)5-aminovaleriansäure
   3-(3,5-Dichlorphenyl)-4-{N'-[5-(4-methyl-pyridin-2-ylamino)-pentanoyl]-hydrazinocarbonyl}-buttersäure, Acetat.
   Nach präparativer HPLC erhält man 3-(3,5-Dichlorphenyl)-4-{N'-[5-(4-methyl-pyridin-2-ylamino)-pentanoyl]-hydrazinocarbonyl}-buttersäure, Trifluoracetat.
   RT = 18,7 (0→60% B, 30 min.)
   MS (ESI): m/z = 482.4 ([M+H]⁺).
2. Analog zu Beispiel 8 erhält man durch Umsetzung von 5-[N'-(9H-Fluoren-9-ylmethoxycarbonyl)-hydrazino]-5-oxo-3-(3,5-dichlorphenyl)-pentansäure mit "DE"
   3-(3,5-Dichlorphenyl)-5-{N'-[1-(3-guanidinophenyl)-methanoyl]-hydrazino}-5-oxo-pentansäure, Trifluoracetat.
   RT = 17,7 (0→60% B, 30 min.)
   MS (ESI): m/z = 453.3 ([M+H]⁺).
3. Analog zu Beispiel 1 erhält man durch Umsetzung von 5-[N'-(9H-Fluoren-9-ylmethoxycarbonyl)-hydrazino]-5-oxo-3-(3,5-dichlorphenyl)-pentansäure mit 3-(9H-Fluoren-9-ylmethoxycarbonylamino)-benzoesäure und Thioacetimidsäure-naphthalin-2-ylmethylester
   5-{N'-[1-(3-Acetimidoylamino-phenyl)-methanoyl]-hydrazino}-3(3,5-dichlorphenyl)-5-oxo-pentansäure, Trifluoracetat.
   RT = 16,6 (0→60% B, 30 min.)
   MS (ESI): m/z = 452.3 ([M+H]⁺).
4. Analog zu Beispiel 9 erhält man durch Umsetzung von 5-[N'-(9H-Fluoren-9-ylmethoxycarbonyl)-hydrazino]-5-oxo-3-(3,5-dichlorphenyl)-pentansäure
   mit 3-(Pyridin-2-ylamino)-benzoesäure
   3-(3,5-Dichlorphenyl)-5-oxo-5-{N'-[3-(pyridin-2-ylamino)-benzoyl]-hydrazino}-pentansäure, Acetat.
   Nach präparativer HPLC erhält man 3-(3,5-Dichlorphenyl)-5-oxo-5-{N'-[3-(pyridin-2-ylamino)-benzoyl]-hydrazino}-pentansäure, Trifluoracetat.
   RT = 33,5 (0→60% B, 30 min.)
   MS (ESI): m/z = 488.3 ([M+H]⁺).
5. Analog zu Beispiel 3 erhält man durch Umsetzung von 5-[N'-(9H-Fluoren-9-ylmethoxycarbonyl)-hydrazino]-5-oxo-3-(3,5-dichlorphenyl)-pentansäure
   mit 3-(9H-Fluoren-9-ylmethoxycarbonylamino)-valeriansäure und "DE"
   3-(3,5-Dichlorphenyl)-5-[N'-(5-guanidino-pentanoyl)-hydrazino]-5-oxopentansäure, Trifluoracetat.
6. Analog zu Beispiel 9 erhält man durch Umsetzung von 5-[N'-(9H-Fluoren-9-ylmethoxycarbonyl)-hydrazino]-5-oxo-3-(3,5-dichlorphenyl)-pentansäure
   mit 4-(Pyridin-2-ylamino)-buttersäure
   3-(3,5-Dichlorphenyl)-5-oxo-5-{N'-[4-(pyridin-2-ylamino)-butanoyl]-hydrazino}-pentansäure, Acetat.
   Nach präparativer HPLC erhält man 3-(3,5-Dichlorphenyl)-5-oxo-5-{N'-[4-(pyridin-2-ylamino)-butanoyl]-hydrazino}-pentansäure, Trifluoracetat.
7. Analog zu Beispiel 9 erhält man durch Umsetzung von 5-[N'-(9H-Fluoren-9-ylmethoxycarbonyl)-hydrazino]-5-oxo-3-(3,5-dichlorphenyl)-pentansäure mit 4-(1H-lmidazol-2-ylamino)-buttersäure
   3-{3,5-Dichlorphenyl)-5-oxo-5-{N'-[4-(1 H-imidazol-2-ylamino)-butanoyl]-hydrazino}-pentansäure, Acetat.
   Nach präparativer HPLC erhält man 3-(3,5-Dichlorphenyl)-5-oxo-5-{N'-[4-(1 H-imidazol-2-ylamino)-butanoy)]-hydrazino}-pentansäure, Trifluoracetat. MS (ESI): m/z = 443.3 ([M+H]⁺).

### Beispiel 19:

1. Analog zu Beispiel 9 erhält man durch Umsetzung von 5-[N'-(9H-Fluoren-9-ylmethoxycarbonyl)-hydrazino]-5-oxo-3-(biphenyl-4-yl)-pentansäure, hergestellt durch Umsetzung von 3-(Biphenyl-4-yl)-glutarsäureanhydrid mit Fmoc-Hydrazin,
   mit N-(2-(4-Methylpyridyl)5-aminovaleriansäure
   3-(Biphenyl-4-yl)-4-{N'-[5-(4-methyl-pyridin-2-ylamino)-pentanoyl]-hydrazinocarbonyl}-buttersäure, Acetat.
   RT = 20,3 (0→60% B, 30 min.)
   MS (ESI): m/z = 489.6 ([M+H]⁺).
2. Analog zu Beispiel 8 erhält man durch Umsetzung von 5-[N'-(9H-Fluoren-9-ylmethoxycarbonyl)-hydrazino]-5-oxo-3-(biphenyl-4-yl)-pentansäure mit "DE"
   3-Biphenyl-4-yl-5-{N'-[1-(3-guanidinophenyl)-methanoyl]-hydrazino}-5-oxo-pentansäure, Trifluoracetat.
   RT = 19,3 (0→60% B, 30 min.)
   MS (ESI): m/z = 460.5 ([M+H]⁺).
3. Analog zu Beispiel 1 erhält man durch Umsetzung von 5-[N'-(9H-Fluoren-9-ylmethoxycarbonyl)-hydrazino]-5-oxo-3-(biphenyl-4-yl)-pentansäure mit 3-(9H-Fluoren-9-ylmethoxycarbonylamino)-benzoesäure und Thioacetimidsäure-naphthalin-2-ylmethylester
   5-{N'-[1-(3-Acetimidoylamino-phenyl)-methanoyl]-hydrazino}-3-biphenyl-4-yl-5-oxo-pentansäure, Trifluoracetat.
   RT = 19,6 (0→60% B, 30 min.)
   MS (ESI): m/z = 459.5 ([M+H]⁺).

### Beispiel 20:

1. Analog zu Beispiel 9 erhält man durch Umsetzung von 5-[N'-(9H-Fluoren-9-ylmethoxycarbonyl)-hydrazino]-5-oxo-3-(3,5-dichlor-2-hydroxyphenyl)-pentansäure, hergestellt durch Umsetzung von 3-(3,5-Dichlor-2-hydroxy-phenyl)-glutarsäureanhydrid mit Fmoc-Hydrazin,
   mit 4-(Pyridin-2-ylamino)-buttersäure
   3-(3,5-Dichlor-2-hydroxy-phenyl)-5-oxo-5-{N'-[4-(pyridin-2-ylamino)-butanoyl]-hydrazino}-pentansäure, Acetat.
   Nach präparativer HPLC erhält man 3-(3,5-Dichlor-2-hydroxy-phenyl)-5-oxo-5-{N'-[4-(pyridin-2-ylamino)-butanoyl]-hydrazino}-pentansäure, Trifluoracetat.
   MS (ESI): m/z = 470.3 ([M+H]⁺).
2. Analog zu Beispiel 9 erhält man durch Umsetzung von 5-[N'-(9H-Fluoren-9-ylmethoxycarbonyl)-hydrazino]-5-oxo-3-(3,5-dichlor-2-hydroxyphenyl)-pentansäure
   mit 4-(1 H-Imidazol-2-ylamino)-buttersäure
   3-(3,5-Dichlor-2-hydroxy-phenyl)-5-oxo-5-(N'-[4-(1 H-imidazol-2-ylamino)-butanoyl]-hydrazino}-pentansäure, Acetat.
   Nach präparativer HPLC erhält man 3-(3,5-Dichlor-2-hydroxy-phenyl)-5-oxo-5-{N'-[4-(1H-imidazol-2-ylamino)-butanoyl]-hydrazino}-pentansäure, Trifluoracetat.
   MS (ESI): m/z = 459.3 ([M+H]⁺).

### Beispiel 21:

1. Analog zu Beispiel 9 erhält man durch Umsetzung von 5-[N'-(9H-Fluoren-9-ylmethoxycarbonyl)-hydrazino]-5-oxo-3-(3-nitrophenyl)-pentansäure, hergestellt durch Umsetzung von 3-(3-Nitrophenyl)-glutarsäureanhydrid mit Fmoc-Hydrazin,
   mit N-(2-(4-Methylpyridyl)5-aminovaleriansäure
   3-(3-Nitrophenyl)-4-{N'-[5-(4-methyl-pyridin-2-ylamino)-pentanoyl]-hydrazinocarbonyt}-buttersäure, Acetat.
   MS (ESI): m/z = 458.5 ([M+H]⁺).
2. Analog zu Beispiel 8 erhält man durch Umsetzung von 5-[N'-(9H-Fluoren-9-ylmethoxycarbonyl)-hydrazino]-5-oxo-3-(3-nitrophenyl)-pentansäure mit "DE"
   5-{N'-[1-(3-Guanidinophenyl)-methanoyl]-hydrazino}-3-(3-nitrophenyl)-5-oxo-pentansäure, Trifluoracetat.
   RT = 11,3 (0→60% B, 30 min.)
   MS (ESI): m/z = 429.4 ([M+H]⁺).
3. Analog zu Beispiel 1 erhält man durch Umsetzung von 5-[N'-(9H-Fluoren-9-ylmethoxycarbonyl)-hydrazino]-5-oxo-3-(3-nitrophenyl)-pentansäure mit 3-(9H-Fluoren-9-ylmethoxycarbonylamino)-benzoesäure und Thioacetimidsäure-naphthalin-2-ylmethylester
   5-{N'-[1-(3-Acetimidoylamino-phenyl)-methanoyl]-hydrazino}-3-(3-nitrophenyl)-5-oxo-pentansäure, Trifluoracetat.

### Beispiel 22:

1. Analog zu Beispiel 9 erhält man durch Umsetzung von 5-[N'-(9H-Fluoren-9-ylmethoxycarbonyl)-hydrazino]-5-oxo-3-(4-methoxyphenyl)-pentansäure, hergestellt durch Umsetzung von 3-(4-Methoxyphenyl)-glutarsäureanhydrid mit Fmoc-Hydrazin,
   mit N-(2-(4-Methylpyridyl)5-aminovaleriansäure
   3-(4-Methoxyphenyl)-4-{N'-[5-(4-methyl-pyridin-2-ylamino)-pentanoyl]-hydrazinocarbonyl}-buttersäure, Acetat.
   RT = 13,7 (0→60% B, 30 min.)
   MS (ESI): m/z = 443.4 ([M+H]⁺).
2. Analog zu Beispiel 8 erhält man durch Umsetzung von 5-[N'-(9H-Fluoren-9-ylmethoxycarbonyl)-hydrazino]-5-oxo-3-(4-methoxyphenyl)-pentansäure mit "DE"
   5-{N'-[1-(3-Guanidinophenyl)-methanoyl]-hydrazino}-3-(4-methoxyphenyl)-5-oxo-pentansäure, Trifluoracetat.
   RT = 10,6 (0→60% B, 30 min.)
   MS (ESI): m/z = 414.4 ([M+H]⁺).
3. Analog zu Beispiel 1 erhält man durch Umsetzung von 5-[N'-(9H-Fluoren-9-ylmethoxycarbonyl)-hydrazino]-5-oxo-3-(4-methoxyphenyl)-pentansäure mit 3-(9H-Fluoren-9-ylmethoxycarbonylamino)-benzoesäure und Thioacetimidsäure-naphthalin-2-ylmethylester
   5-{N'-[1-(3-Acetimidoylamino-phenyl)-methanoyl]-hydrazino}-3-(4-methoxyphenyl)-5-oxo-pentansäure, Trifluoracetat.
   RT = 10,5 (0→60% B, 30 min.)
   MS (ESI): m/z = 413.4 ([M+H]⁺).

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 1 isotonischer NaCI-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 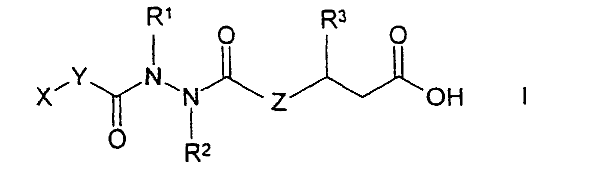 worin
X H₂N-C(=NH)-NH-, H₃C-C(=NH)-NH oder Het¹-NH-,
Y -(CH₂)ₙ- oder 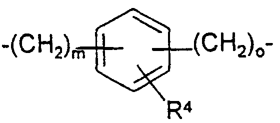
Z N-R² oder CH-R²,
R¹, R² jeweils unabhängig voneinander H oder A,
R³ H, Ar oder Het,
R⁴ H, A, Ar, OH, OA, OAr, Arylalkyl, Hal, CN, NO₂, CF₃ oder OCF₃,
A Alkyl mit 1-6 C-Atomen,
Ar unsubstituiertes oder ein-, zwei- oder dreifach durch A, OH, OA, CF₃, OCF₃, CN, NO₂ oder Hal substituiertes Phenyl, welches durch ein ein-, zwei- oder dreifach durch A, OH, OA, CF₃, OCF₃, CN, NO₂ oder Hal substituiertes Phenyl in der Art substituiert sein kann, daß ein unsubstituiertes oder substituiertes Biphenyl entsteht oder unsubstituiertes oder ein-, zwei- oder dreifach durch A, OH, OA, CF₃, OCF₃, CN, NO₂ oder Hal substituiertes Naphthyl,
Hal F, Cl, Br oder I,
Het einen gesättigten, teilweise oder vollständig ungesättigten mono- oder bicyclischen heterocyclischen Rest mit 5 bis 10 Ringgliedern, wobei 1 oder 2 N- und/oder 1 oder 2 S- oder O- Atome vorliegen können und der heterocyclische Rest ein-oder zweifach durch CN, Hal, OH, OA, CF₃, A, NO₂ oder OCF₃ substituiert sein kann,
Het¹ einen 5- oder 6-gliedrigen aromatischen Heterocyclus mit 1 bis 4 N-Atomen, der unsubstituiert oder ein- oder zweifach durch Hal, A, OA, Ar, OAr, Arylalkyl, CN, NO₂, CF₃ oder OCF₃ substituiert sein kann,
n 2, 3, 4, 5 oder 6,
m, o 0, 1 oder 2
bedeuten,
sowie ihre physiologisch unbedenklichen Salze und Solvate.

2. Verbindungen der Formel I gemäß Anspruch 1
a) 5-[N'-(3-Guanidino-benzoyl)-hydrazino]-5-oxo-3-phenyl-pentansäure,
b) 3-[4-(3-Guanidino-phenylacetyl)-semicarbazido]-3-phenylpropionsäure,
c) 3-[4-(3-Guanidinobenzoyl)-semicarbazido]-3-phenyl-propionsäure,
d) 4-{N'-[5-(4-Methyl-pyridin-2-ylamino)-pentanoyl]-hydrazinocarbonyl}-3-phenyl-buttersäure,
e) 3-[4-(5-Guanidino-pentanoyl)-semicarbazido]-3-phenyl-propionsäure,
f) 4-[N'-(5-Guanidino-pentanoyl)-hydrazinocarbonyl]-3-phenylbuttersäure,
g) 3-[4-(3-Guanidinobenzoyl)-semicarbazido]-3-(4-chlorphenyl)-propionsäure,
h) 3-{4-[5-(4-Methyl-pyridin-2-ylamino)-pentanoyl]-semicarbazido}-3-(4-chlorphenyl)-propionsäure,
i) 3-[4-(5-Guanidinopentanoyl)-semicarbazidol-3-(4-chlorphenyl)-propionsäure,
sowie ihre physiologisch unbedenklichen Salze oder Solvate.

3. Verfahren zur Herstellung von Verbindungen der Formel I nach Anspruch 1 sowie ihrer Salze, **dadurch gekennzeichnet, daß** man
a) zur Herstellung einer Verbindung der Formel I, worin Z N-R² bedeutet, eine Verbindung der Formel II, 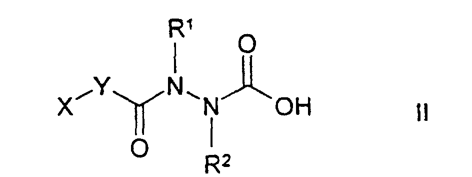 worin X, Y, R¹ und R² die in Anspruch 1 angegebenen Bedeutungen haben, und worin freie Aminogruppen durch eine geeignete Aminoschutzgruppe geschützt sind,
mit einer Verbindung der Formel III 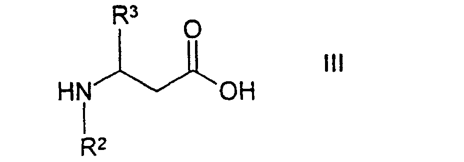 worin R² und R³ die in Anspruch 1 angegebenen Bedeutungen haben und worin eine freie Hydroxylgruppe durch eine geeignete Hydroxylschutzgruppe geschützt ist oder an eine feste Phase gebunden ist,
umsetzt,
und anschließend die Schutzgruppen und/oder die feste Phase entfernt,
oder
b) eine Verbindung der Formel IV, 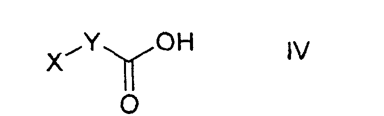 worin X und Y die in Anspruch 1 angegebenen Bedeutungen haben, und worin freie Aminogruppen durch eine geeignete Aminoschutzgruppe geschützt sind,
mit einer Verbindung der Formel V 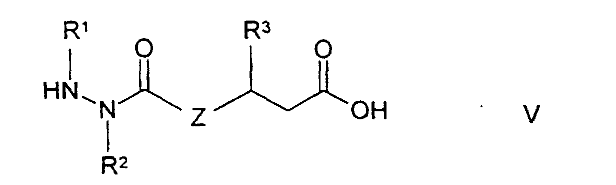 worin R¹, R², R³ und Z die in Anspruch 1 angegebenen Bedeutungen haben, und worin eine freie Hydroxylgruppe durch eine geeignete Hydroxylschutzgruppe geschützt ist oder an eine feste Phase gebunden ist,
umsetzt,
und anschließend die Schutzgruppen und/oder die feste Phase entfernt;
oder
c) eine Verbindung der Formel I aus einem ihrer funktionellen Derivate durch Behandeln mit einem solvolysierenden oder
hydrogenolysierenden Mittel in Freiheit setzt,
und/oder daß man eine basische oder saure Verbindung der Formet I durch Behandeln mit einer Säure oder Base in eines ihrer Salze
überführt.

4. Verbindungen der Formel I nach Anspruch 1 und ihre physiologisch unbedenklichen Salze oder Solvate als therapeutische Wirkstoffe.

5. Verbindungen der Formel I nach Anspruch 1 und ihre physiologisch unbedenklichen Salze oder Solvate als Integrininhibitoren.

6. Verbindungen der Formel I nach Anspruch 1 und ihre physiologisch unbedenklichen Salze oder Solvate zur Anwendung bei der Bekämpfung von Krankheiten.

7. Pharmazeutische Zubereitung **gekennzeichnet** duch einen Gehalt an mindestens einer Verbindung der Formel I nach Anspruch 1 und/oder einem ihrer physiologisch unbedenklichen Salze oder Solvate.

8. Verwendung von Verbindungen der Formel I nach Anspruch 1 und/oder ihre physiologisch unbedenklichen Salze oder Solvate zur Herstellung einer pharmazeutischen Zubereitung.

9. Verwendung von Verbindungen der Formel I nach Anspruch 1 und/oder ihrer physiologisch unbedenklichen Salze oder Solvate zur Herstellung einer pharmazeutischen Zubereitung zur Bekämpfung von Thrombosen, Herzinfarkt, koronaren Herzerkrankungen, Arteriosklerose, Entzündungen, Tumoren, Osteoporose, Infektionen und Restenose nach Angioplastie.

10. Verwendung von Verbindungen der Formel I nach Anspruch 1 und/oder ihre physiologisch unbedenklichen Salze oder Solvate zur Herstellung einer pharmazeutischen zubereitung verwendbar bei pathologischen Vorgängen, die durch Angiogenese unterhalten oder propagiert werden.

## Claims

1. Compounds of the formula I 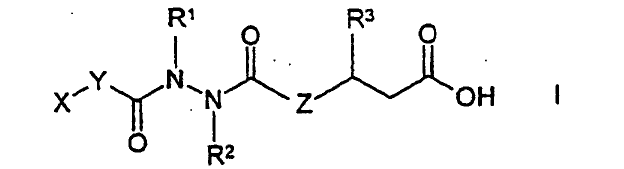 in which
X is H₂N-C(=NH)-NH-, H₃C-C(=NH)-NH or Het¹-NH-,
Y is -(CH₂)ₙ- or 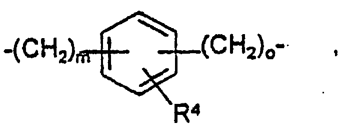
Z is N-R² or CH-R²,
R¹ and R² are each, independently of one another, H or A,
R³ is H, Ar or Het,
R⁴ is H, A, Ar, OH, OA, OAr, arylalkyl, Hal, CN, NO₂, CF₃ or OCF3,
A is alkyl having 1-6 carbon atoms,
Ar is phenyl which is unsubstituted or monosubstituted, disubstituted or trisubstituted by A, OH, OA, CF₃, OCF₃, CN, NO₂ or Hal and which may be substituted by phenyl which is monosubstituted, disubstituted or trisubstituted by A, OH, OA, CF₃, OCF₃, CN, NO₂ or Hal so as to give an unsubstituted or substituted biphenyl, or naphthyl which is unsubstituted or monosubstituted, disubstituted or trisubstituted by A, OH, OA, CF₃, OCF₃, CN, NO₂ or Hal,
Hal is F, Cl, Br or I,
Het is a saturated, partially or fully unsaturated monocyclic or bicyclic heterocyclic radical having from 5 to 10 ring members, where 1 or 2 N atoms and/or 1 or 2 S or O atoms may be present, and the heterocyclic radical may be monosubstituted or disubstituted by CN, Hal, OH, OA, CF₃, A, NO₂ or OCF₃,
Het¹ is a 5- or 6-membered aromatic heterocyclic radical having from 1 to 4 N atoms, which may be unsubstituted or monosubstituted or disubstituted by Hal, A, OA, Ar, OAr, arylalkyl, CN, NO₂, CF₃ or OCF₃,
n is 2, 3, 4, 5 or 6,
m and o are 0.1 or 2,
and physiologically acceptable salts and solvates thereof.

2. Compounds of the formula I according to Claim 1
a) 5-[N'-(3-guanidinobenzoyl)hydrazino]-5-oxo-3-phenylpentanoic acid,
b) 3-[4-(3-guanidinophenylacetyl)semicarbazido]-3-phenylpropionic acid,
c) 3-[4-(3-guanidinobenzoyl)semicarbazido]-3-phenylpropionic acid,
d) 4-{N'-[5-(4-methylpyridin-2-ylamino)pentanoyl]hydrazinocarbonyl}-3-phenylbutyric acid,
e) 3-[4-(5-guanidinopentanoyl)semicarbazido]-3-phenylpropionic acid,
f) 4-[N'-(5-guanidinopentanoyl)hydrazinocarbonyl]-3-phenylbutyric acid,
g) 3-[4-(3-guanidinobenzoyl)semicarbazido]-3-(4-chlorophenyl)-propionic acid,
h) 3-{4-[5-(4-methylpyridin-2-ylamino)pentanoyl]semicarbazido}-3-(4-chlorophenyl)propionic acid,
i) 3-[4-(5-guanidinopentanoyl)semicarbazido]-3-(4-chlorophenyl)-propionic acid,
and physiologically acceptable salts or solvates thereof.

3. Process for the preparation of compounds of the formula I according to Claim 1 and salts thereof, **characterised in that**
a) for the preparation of a compound of the formula I in which Z is N-R², a compound of the formula II 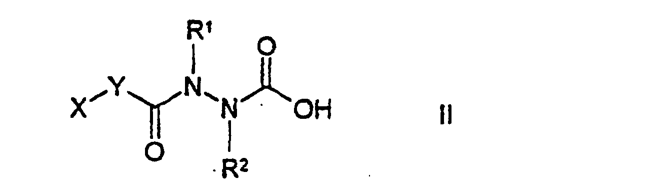 in which X, Y, R¹ and R² are as defined in Claim 1, and in which free amino groups are protected by a suitable amino-protecting group,
is reacted with a compound of the formula III 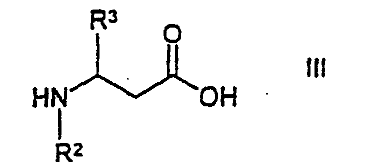 in which R² and R³ are as defined in Claim 1 and in which a free hydroxyl group is protected by a suitable hydroxyl-protecting group or is bonded to a solid phase,
and subsequently the protecting groups and/or the solid phase are removed,
or
b) a compound of the formula IV 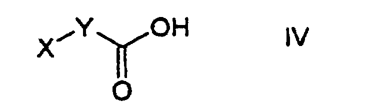 in which X and Y are as defined in Claim 1, and in which free amino groups are protected by a suitable amino-protecting group,
is reacted with a compound of the formula V 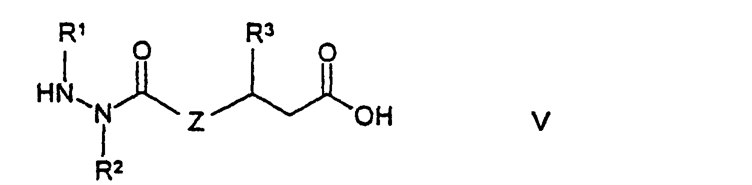 in which R¹, R², R³ and Z are as defined in Claim 1, and in which a free hydroxyl group is protected by a suitable hydroxyl-protecting group or is bonded to a solid phase,
and subsequently the protecting groups and/or the solid phase are removed;
or
c) a compound of the formula I is liberated from one of its functional derivatives by treatment with a solvolysing or hydrogenolysing agent,
and/or **in that** a basic or acidic compound of the formula I is converted into one of its salts by treatment with an acid or base.

4. Compounds of the formula I according to Claim 1 and physiologically acceptable salts or solvates thereof as therapeutic active ingredients.

5. Compounds of the formula I according to Claim 1 and physiologically acceptable salts or solvates thereof as integrin inhibitors.

6. Compounds of the formula I according to Claim 1 and physiologically acceptable salts or solvates thereof for use in combating diseases.

7. Pharmaceutical preparation, **characterised by** a content of at least one compound of the formula I according to Claim 1 and/or one of its physiologically acceptable salts or solvates.

8. Use of compounds of the formula I according to Claim 1 and/or physiologically acceptable salts or solvates thereof for the preparation of a pharmaceutical preparation.

9. Use of compounds of the formula I according to Claim 1 and/or physiologically acceptable salts or solvates thereof for the preparation of a pharmaceutical preparation for combating thromboses, cardiac infarction, coronary heart diseases, arteriosclerosis, inflammation, tumours, osteoporosis, infections and restenosis after angioplasty.

10. Use of compounds of the formula I according to Claim 1 and/or physiologically acceptable salts or solvates thereof for the preparation of a pharmaceutical preparation which can be used in pathological processes which are maintained or propagated by angiogenesis.

## Revendications

1. Composés de formule I 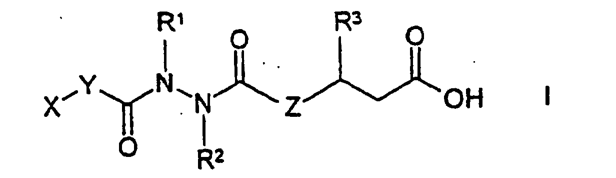 dans laquelle
X est H₂N-C(=NH)-NH-, H₃C-C(=NH)-NH ou Hét¹-NH-,
Y est -(CH₂)ₙ- ou 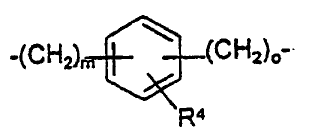
Z est N-R² ou CH-R²,
R¹ et R² sont chacun, indépendamment l'un de l'autre, H ou A,
R³ est H, Ar ou Hét,
R⁴ est H, A, Ar, OH, OA, OAr, arylalkyle, Hal, CN, NO₂, CF₃ ou OCF₃,
A est alkyle ayant de 1 à 6 atomes de carbone,
Ar est phényle qui est non substitué ou monosubstitué, disubstitué ou trisubstitué par A, OH, OA, CF₃, OCF₃, CN, NO₂ ou Hal et qui peut être substitué par phényle qui est monosubstitué, disubstitué ou trisubstitué par A, OH, OA, CF₃, OCF₃, CN, NO₂ ou Hal de façon à donner biphényle non substitué ou substitué, ou naphtyle qui est non substitué ou monosubstitué, disubstitué ou trisubstitué par A, OH, OA, CF₃, OCF₃, CN, NO₂ ou Hal,
Hal est F, Cl, Br ou I,
Hét est un radical hétérocyclique monocyclique ou bicyclique saturé ou partiellement ou totalement insaturé, ayant de 5 à 10 chaînons, dans lequel 1 ou 2 atomes de N et/ou 1 ou 2 atomes de S ou de O peuvent être présents, et le radical hétérocyclique peut être monosubstitué ou disubstitué par CN, Hal, OH, OA, CF₃, A, NO₂ ou OCF₃,
Hét¹ est un radical hétérocyclique aromatique à 5 ou 6 chaînons ayant de 1 à 4 atomes de N, qui peut être non substitué ou monosubstitué ou disubstitué par Hal, A, OA, Ar, OAr, arylalkyle, CN, NO₂, CF₃ ou OCF₃,
n vaut 2, 3, 4, 5 ou 6,
m et o valent 0, 1 ou 2,
et les sels et solvats physiologiquement acceptables de ceux-ci.

2. Composés de formule I selon la revendication 1
a) l'acide 5-[N'-(3-guanidinobenzoyl)hydrazino]-5-oxo-3-phényl-pentanoïque,
b) l'acide 3-[4-(3-guanidinophénylacétyl)semicarbazido]-3-phényl-propionique,
c) l'acide 3-[4-(3-guanidinobenzoyl)semicarbazido]-3-phényl-propionique,
d) l'acide 4-{N'-[5-(4-méthylpyridin-2-ylamino)pentanoyl]hydrazino-carbonyl}-3-phénylbutyrique,
e) l'acide 3-[4-(5-guanidinopentanoyl)semicarbazido]-3-phényl-propionique,
f) l'acide 4-[N'-(5-guanidinopentanoyl)hydrazinocarbonyl]-3-phényl-butyrique,
g) l'acide 3-[4-(3-guanidinobenzoyl)semicarbazido]-3-(4-chlorophényl)-propionique,
h) l'acide 3-{4-[5-(4-méthylpyridin-2-ylamino)pentanoyl]semicarbazido}-3-(4-chlorophényl)propionique,
i) l'acide 3-[4-(5-guanidinopentanoyl)semicarbazido]-3-(4-chloro-phényl)propionique,
et les sels ou solvats physiologiquement acceptables de ceux-ci.

3. Procédé de préparation de composés de formule I selon la revendication 1 et de sels de ceux-ci, **caractérisé en ce que**
a) pour la préparation d'un composé de formule I dans laquelle Z est N-R²,
un composé de formule II 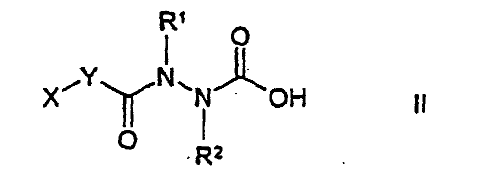 dans laquelle X, Y, R¹ et R² sont tels que définis selon la revendication 1, et dans laquelle les groupements amino libres sont protégés par un groupement protecteur d'amino convenable,
est réagi avec un composé de formule III 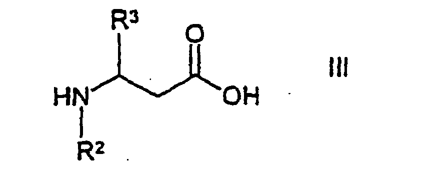 dans laquelle R² et R³ sont tels que définis selon la revendication 1 et dans laquelle un groupement hydroxyle libre est protégé par un groupement protecteur d'hydroxyle convenable ou est lié à une phase solide,
et ensuite les groupements protecteurs et/ou la phase solide sont éliminés, .
ou
b) un composé de formule IV 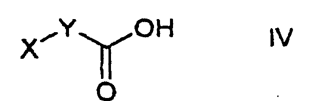 dans laquelle X et Y sont tels que définis dans la revendication 1, et dans laquelle les groupements amino libres sont protégés par un groupement protecteur d'amino convenable,
est réagi avec un composé de formule V 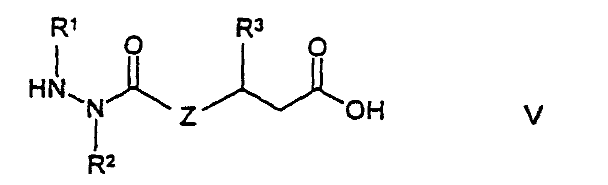 dans laquelle R¹, R², R³ et Z sont tels que définis selon la revendication 1, et dans laquelle un groupement hydroxyle libre est protégé par un groupement protecteur d'hydroxyle convenable ou est lié à une phase solide,
et ensuite les groupements protecteurs et/ou la phase solide sont éliminés ;
ou
c) un composé de formule I est libéré depuis l'un de ses dérivés fonctionnels par un traitement par un agent de solvolysation ou d'hydrogénolysation,
et/ou **en ce qu'**un composé basique ou acide de formule I est converti en l'un de ses sels par un traitement par un acide ou une base.

4. Composés de formule I selon la revendication 1 et les sels ou solvats physiologiquement acceptables de ceux-ci comme ingrédients thérapeutiquement actifs.

5. Composés de formule I selon la revendication 1 et les sels ou solvats physiologiquement acceptables de ceux-ci comme inhibiteurs de l'intégrine.

6. Composés de formule I selon la revendication 1 et les sels ou solvats physiologiquement acceptables de ceux-ci pour une utilisation dans la lutte contre des maladies.

7. Préparation pharmaceutique, **caractérisée par** une teneur en au moins un composé de formule I selon la revendication 1 et/ou l'un de ses sels ou solvats physiologiquement acceptables.

8. Utilisation de composés de formule I selon la revendication 1 et/ou de sels ou solvats physiologiquement acceptables de ceux-ci pour la préparation d'une préparation pharmaceutique.

9. Utilisation de composés de formule I selon la revendication 1 et/ou de sels ou solvats physiologiquement acceptables de ceux-ci pour la préparation d'une préparation pharmaceutique pour lutter contre les thromboses, l'infarctus du myocarde, les maladies coronariennes, l'artériosclérose, les inflammations, les tumeurs, l'ostéoporose, les infections et la resténose post-angioplastie.

10. Utilisation de composés de formule I selon la revendication 1 et/ou de sels ou solvats physiologiquement acceptables de ceux-ci pour la préparation d'une préparation pharmaceutique pouvant être utilisée dans des procédés pathologiques qui sont maintenus ou propagés par angiogenèse.
